(19) Europäisches Patentamt
European Patent Office
Office européen des brevets

(11) Veröffentlichungsnummer: **0 212 481**
**B1**

(12) # EUROPÄISCHE PATENTSCHRIFT

(45) Veröffentlichungstag der Patentschrift: **12.09.90**

(21) Anmeldenummer: **86110952.8**

(22) Anmeldetag: **08.08.86**

(51) Int. Cl.[5]: **C 07 D 209/34,**
A 61 K 31/445,
A 61 K 31/495, C 07 D 401/12
// (C07D401/12, 309:34,
211:36)

(54) **Indolinonderivate, Verfahren zu ihrer Herstellung, sie enthaltende Arzneimittel und deren Verwendung.**

(30) Priorität: **22.08.85 DE 3529994**

(43) Veröffentlichungstag der Anmeldung:
**04.03.87 Patentblatt 87/10**

(45) Bekanntmachung des Hinweises auf die Patenterteilung:
**12.09.90 Patentblatt 90/37**

(84) Benannte Vertragsstaaten:
**AT BE CH DE FR GB IT LI LU NL SE**

(56) Entgegenhaltungen:
**EP-A-0 116 368**
**EP-A-0 146 893**
**DE-A-2 451 592**
**DE-A-2 533 863**
**DE-B-2 521 966**

(73) Patentinhaber: **HOECHST AKTIENGESELLSCHAFT**
**Postfach 80 03 20**
**D-6230 Frankfurt am Main 80 (DE)**

(72) Erfinder: **Lerch, Ulrich, Dr.**
**Vorderwart 24**
**D-6238 Hofheim am Taunus (DE)**
Erfinder: **Henning, Rainer, Dr.**
**Rotenhofstrasse 31**
**D-6234 Hattersheim am Main (DE)**
Erfinder: **Kaiser, Joachim, Dr.**
**Fichtestrasse 12**
**D-6000 Frankfurt am Main (DE)**

**EP 0 212 481 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Es ist bekannt, daß Verbindungen, die das Einströmen von Calcium-Ionen in Zellen behindern, als Therapeutika zur Behandlung von verschiedenen Krankheiten, insbesondere das Herz-Kreislauf-Systems beim Menschen und anderen Warmblütern eingesetzt werden können.

Die Synthese von 3-Arylindolin-2-onen ist bekannt; nicht bekannt sind jedoch durch eine basische Ethergruppierung am 3-Phenylrest substituierte 3-Aryl-indolin-2-one; nicht bekannt sind ferner solche Indolinon-Verbindungen mit calcium-antagonistischer Wirkung.

Die EP—A—116 368 beschreibt Benzo-thiazinon-Derivate mit Calcium-antagonistischer Wirkung; Verbindungen mit einer Indolinon-Grundstruktur sind jedoch durch diese Publikation weder vorweggenommen noch nahegelegt.

Die EP—A—146 893 beschreibt ebenfalls Benzothiazin-Derivate mit Calcium-antagonistischer Wirksamkeit, jedoch sind wiederum Indolinone weder beschreiben noch nahegelegt.

Die Erfindung betrifft Indolinonderivate der Formel I, die eine solche Wirkung aufweisen,

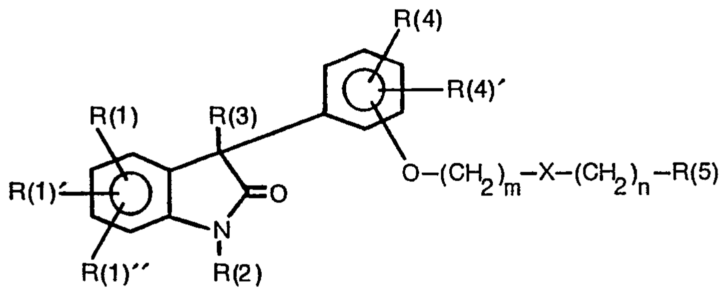

(I)

und in welcher

R(1), R(1)' und R(1)'' gleich oder verschieden und voneinander unabhängig sind und Wasserstoff, $(C_1—C_4)$-Alkyl, $(C_1—C_3)$-Alkoxy, F, Cl, Br, $CF_3$, Nitro, Hydroxy, Acetamido oder Amino,

R(2) Wasserstoff, $(C_1—C_{10})$-Alkyl, geradkettig oder verzsweigt, $(C_3—C_{10})$-Alkenyl, geradkettig oder verzweigt, Phenyl-$(C_1—C_4)$-alkyl, wobei der Phenylring unsubstituiert oder durch einen, zwei oder drei Substituenten der Gruppe $(C_1—C_4)$-Alkyl, $(C_1—C_3)$-Alkoxy, F, Cl, $CF_3$, $(C_1—C_2)$-Alkylendioxy oder Nitro substituiert ist,

R(3) Wasserstoff, $(C_1—C_{15})$-Alkyl, geradkettig oder verzweigt, $(C_3—C_{15})$-Alkenyl, geradkettig oder verzweigt, $(C_4—C_8)$-Cycloalkyl, $(C_4—C_8)$-Cycloalkyl-$(C_1—C_4)$alkyl, Phenyl oder Phenyl-$(C_1—C_4)$-alkyl, wobei der Phenylrest unsubstituert oder durch einen, zwei oder drei Substituenten der Gruppe $(C_1—C_4)$-Alkyl, $(C_1—C_3)$-Alkoxy, F, Cl, $CF_3$, $(C_1—C_2)$-Alkylendioxy oder Nitro substituiert ist,

R(4) und R(4)' gleich oder verschieden und voneinander unabhängig Wasserstoff, $(C_1—C_4)$-Alkyl, $(C_1—C_3)$-Alkoxy, F, Cl, $CF_3$, Nitro, Hydroxy, Acetamido oder Amino,

m 1, 2, 3 oder 4,

n 0, 1, 2 oder 3, sofern X ein Heteroatom ist, jedoch nur 2 oder 3,

X eine $CH_2$-Gruppe, Sauerstoff, Schwefel, eine Carbonylgruppe, eine CH (OH)-Gruppe oder eine Gruppe

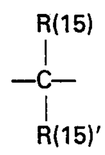

worin

R(15) und R(15)' gleich oder verschieden sind und Wasser-Wasserstoff oder $(C_1—C_4)$-Alkyl bedeuten,

R(5) eine der folgenden Gruppen

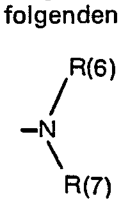

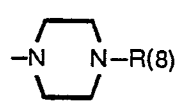

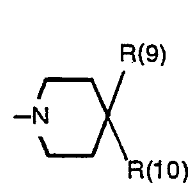

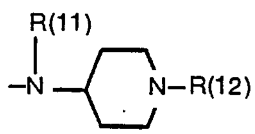

oder

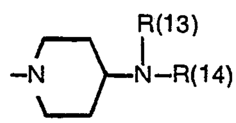

worin

R(6) und R(7) gleich oder verschieden voneinander unabhängig Wasserstoff, $(C_1—C_{10})$-Alkyl, $(C_4—C_8)$-Cycloalkyl, $(C_4—C_8)$-Cycloalkyl-$(C_1—C_4)$-alkyl, Pyridyl-$(C_1—C_4)$-alkyl, Phenyl-$(C_1—C_6)$-alkyl, Benzhydryl oder Benzhydryl-$(C_1—C_4)$-alkyl, wobei die Phenylreste jeweils unsubstituiert oder durch einen, zwei oder drei Reste aus der Gruppe $(C_1—C_4)$-Alkyl, $(C_1—C_4)$-Alkoxy, $(C_1—C_2)$-Alkylendioxy, F, Cl, Br, $CF_3$ oder Hydroxy substituiert sind,

R(8) Wasserstoff, $(C_1—C_{10})$-Alkyl, geradkettig oder verzweigt, $(C_1—C_8)$-Alkanoyl, Pyridyl, Pyrimidinyl, Phenyl, Phenyl-$(C_1—C_4)$-alkyl, Phenyl-$(C_3—C_5)$-alkenyl, Benzhydryl oder Benzhydryl -$(C_1—C_4)$-alkyl, Phenyl-$(C_1—C_4)$-alkanoyl oder Benzoyl, wobei die Phenylreste jeweils unsubstituiert oder durch einen, zwei oder drei Reste aus der Gruppe $(C_1—C_4)$-Alkyl, $(C_1—C_4)$-Alkoxy, $(C_1—C_2)$-Alkylendioxy, F, Cl, Br, $CF_3$ oder Hydroxy substituiert sind,

R(9) Wasserstoff, $(C_1—C_{10})$-Alkyl, Phenyl, Phenyl-$(C_1—C_4)$-alkyl, wobei der Phenylrest jeweils unsubstituiert oder durch einen, zwei oder drei Reste aus der Gruppe $(C_1—C_4)$-Alkyl, $(C_1—C_4)$ Alkoxy, $(C_1—C_2)$-Alkylendioxy, F, Cl, Br, $CF_3$ oder Hydroxy substituiert ist,

R(10) Wasserstoff, Hydroxy, oder $(C_1—C_4)$-Alkoxy, und

R(11) und R(12) bzw. R(13) und R(14) gleich oder verschieden und voneinander unabhängig Wasserstoff, $(C_1—C_{10})$-Alkyl, geradkettig oder verzweigt, $(C_1—C_6)$-Alkanoyl, Phenyl-$(C_1—C_4)$-alkyl, Benzhydryl oder Benzhydryl-$(C_1—C_4)$-alkyl, Phenyl-$(C_1—C_4)$-alkanoyl, oder Benzoyl, wobei die Phenylreste jeweils unsubstituiert oder durch einen, zwei oder drei Reste aus der Gruppe $(C_1—C_4$-Alkyl), $(C_1—C_4)$-Alkoxy, $(C_1—C_2)$-Alkylendioxy, F, Cl, Br, $CF_3$ oder Hydroxy substituiert sind,

bedeuten,

sowie die Salze der Verbindungen der Formel I mit pharmazeutisch akzeptablen Säuren.

Bevorzugt werden die Verbindungen der Formel I, in welcher

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)'' Wasserstoff,

R(2) Wasserstoff, $(C_1—C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1—C_{12})$-Alkyl, geradkettig oder verzweigt, Allyl, Methallyl, $(C_5—C_7)$-Cycloalkyl, $(C_5—C_7)$-Cycloalkyl-$(C_1—C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

m 1, 2, 3 oder 4

n 0, 1, 2 oder 3, sofern X ein Heteroatom bedeutet, jedoch nur 2 oder 3,

X eine $CH_2$-Gruppe, Sauerstoff, Schwefel, eine Carbonylgruppe eine CH(OH)-Gruppe oder eine Gruppe

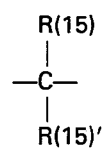

worin

R(15) und R(15)' gleich oder verschieden sind und Wasserstoff, Methyl oder Ethyl bedeuten

R(5) eine der Gruppen

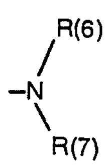

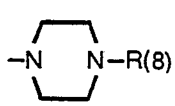

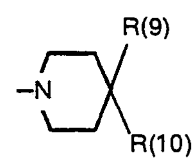

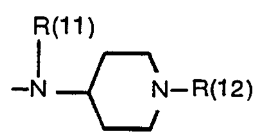

oder

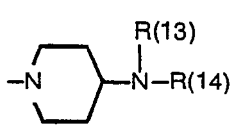

worin

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-$(C_1—C_4)$-alkyl, Benzhydryl oder Benzhydryl-$(C_1—C_4)$-alkyl, wobei die Phenylreste jeweils unsubstituiert oder durch

einen, zwei oder drei Reste aus der Gruppe ($C_1$—$C_4$)-Alkyl, ($C_1$—$C_4$)-Alkoxy, ($C_1$—$C_2$)-Alkylendioxy, F, Cl, $CF_3$ oder Hydroxy substituiert sind, Pyridyl-($C_1$—$C_4$)-alkyl,

R(8) Wasserstoff, ($C_1$—$C_6$)-Alkyl, geradkettig oder verzweigt, ($C_1$—$C_6$)-Alkanoyl, Phenyl, wobei der Phenylrest durch einen oder zwei Reste aus der Gruppe ($C_1$—$C_4$)-Alkyl, ($C_1$—$C_4$)-Alkoxy, ($C_1$—$C_2$)-Alkylendioxy, F, Cl, $CF_3$ oder Hydroxy substituiert sein kann, Phenyl-($C_1$—$C_4$)-alkyl, Phenyl-($C_3$—$C_5$)-alkenyl, Benzhydryl oder Benzhydryl -($C_1$—$C_4$)-alkyl, Phenyl-($C_1$—$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, ($C_1$—$C_2$)-Alkylendioxy, F, Cl, $CF_3$ oder Hydroxy substituiert sind,

R(9) Phenyl, Phenyl-($C_1$—$C_4$)-alkyl, wobei der Phenylrest jeweils unsubstituiert oder durch einen, zwei oder drei Reste aus der Gruppe ($C_1$—$C_4$)-Alkyl, ($C_1$—$C_4$)-Alkoxy, ($C_1$—$C_2$)-Alkylendioxy, F, Cl, $CF_3$ oder Hydroxy substituiert ist,

R(10) Wasserstoff, Hydroxy oder Methoxy

R(11), R(12), R(13) und R(14) gleich oder verschieden und Wasserstoff, ($C_1$—$C_8$)-Alkyl, ($C_1$—$C_6$)-Alkanoyl, Phenyl-($C_1$—$C_4$)-alkyl, Benzhydryl oder Benzhydryl-($C_1$—$C_4$)-alkyl, wobei die Phenylreste jeweils unsubstituiert oder durch einen, zwei oder drei Reste aus der Gruppe Methyl, Ethyl, Methoxy, Ethoxy, ($C_1$—$C_2$)-Alkylendioxy, F, Cl, $CF_3$ oder Hydroxy substituiert sein können, Phenyl-($C_1$—$C_4$)-alkanoyl oder Benzoyl, wobei die Phenylreste jeweils unsubstituiert oder durch einen, zwei oder drei Reste der Gruppe Methyl, Ethyl, Methoxy, Ethoxy, ($C_1$—$C_2$)-Alkylendioxy, F, Cl, $CF_3$ oder Hydroxy substituiert sind, bedeuten,

sowie die Salze dieser Verbindungen der Formel I mit pharmazeutisch akzeptablen Säuren.

Besonders bevorzugt werden Verbindungen der Formel I, in welcher

R(1) Wasserstoff, Methyl, Methoxy, Fluor oder Chlor,

R(1)' Wasserstoff oder Methoxy

R(1)'' Wasserstoff

R(2) Wasserstoff, Methyl, Ethyl, Propyl, Isopropyl, Butyl, sec. Butyl, Isobutyl, Benzyl, Phenethyl,

R(3) Wasserstoff, ($C_1$—$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl

R(4) Wasserstoff, Methoxy, Methyl, Chlor, Nitro oder Hydroxy

R(4)' Wasserstoff

m 1, 2 oder 3

n 0, 1 oder 2, sofern X ein Heteroatom bedeutet, jedoch nur 2

X eine $CH_2$-Gruppe, Sauerstoff, eine Carbonylgruppe, eine CH(OH)-Gruppe oder eine Gruppe

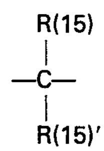

worin

R(15) und R(15)' gleich oder verschieden sind und Wasserstoff oder Methyl bedeuten,

R(5) eine der folgenden Gruppen

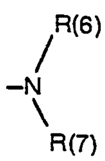

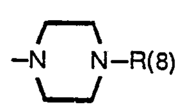

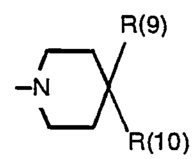

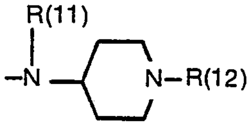

oder

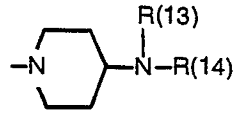

worin

R(6) Wasserstoff oder Methyl

R(7) Phenyl-($C_1$—$C_4$)-alkyl, Benzhydryl oder Benzhydryl-($C_1$—$C_4$) alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist

R(8) ($C_1$—$C_6$)-Alkyl, geradkettig oder verzweigt, ($C_1$—$C_6$)-Alkanoyl, Phenyl, Phenyl-($C_1$—$C_4$)-alkyl, Benzhydryl oder Benzhydryl-($C_1$—$C_4$)-alkyl, Phenyl-($C_1$—$C_4$)-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind

R(9) Phenyl, wobei der Phenylrest unsubstitutiert oder durch einen, zwei oder drei Reste aus der Gruppe Methyl, Methoxy, Fluor, Chlor, Methylendioxy oder Hydroxy substituiert ist

R(10) Wasserstoff, Hydroxy oder Methoxy

R(11), R(12), R(13) und R(14) gleich oder verschieden sind und Wasserstoff, ($C_1$—$C_6$)-Alkyl, ($C_1$—$C_6$)-Alkanoyl, Phenyl-($C_1$—$C_4$)-alkyl, Benzhydryl oder Benzhydryl-($C_1$—$C_4$)-alkyl, Phenyl-($C_1$—$C_4$)-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind,

bedeuten

sowie die Salze dieser Verbindungen der Formel I mit pharmazeutisch akzeptablen Säuren.

Als solche pharmazeutisch akzeptablen Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure, Maleinsäure, Fumarsäure, Malonsäure, Oxalsäure, Gluconsäure, Camphersulfonsäure, Benzolsulfonsäure, Essigsäure, Propionsäure oder p-Toluol- sulfonsäure in Betracht.

Die Verbindungen der Formel I weisen asymmetrische C-Atome auf und können daher als Enantiomere oder Diastereomere auftreten. Die Erfindung umfaßt sowohl die reinen Isomeren als auch deren Gemische. Diese Gemische von Diastereomeren können nach gebräuchlichen Methoden, zum Beispiel selektive Kristallisation aus geeigneten Lösungsmitteln oder Chromatographie an Kieselgel oder Aluminiumoxid in die Komponeten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin Verfahren zur Herstellung von Verbindungen der Formel I, dadurch gekennzeichnet, daß man

a) eine Verbindung der Formel II,

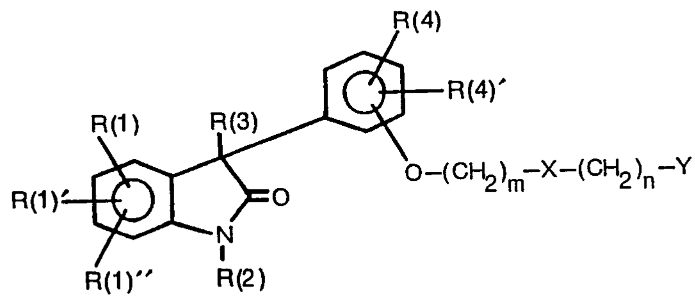

(II)

in welcher R(1), R(1)', R(1)'', R(2), R(3), R(4), R(4)', X, m und n die gleiche Bedeutung wie in Formel I haben, und in welcher Y eine Abgangsgruppe, die nucleophil verdrängt werden kann, insbesondere ein Chlor-, Brom- oder Jodatom, einen Sulfonsäurerest, vorzugsweise einen Methansulfonylrest, einen Benzolsulfonylrest, einen Toluolsulfonylrest oder einen Trifluormethansulfonylrest bedeutet, mit einer der Verbindungen der Formeln IIIa, IIIb, IIIc, IIId oder IIIe

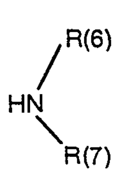

IIIa

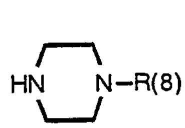

IIIb

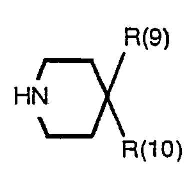

IIIc

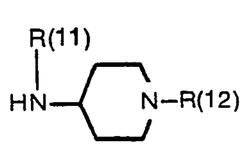

IIId

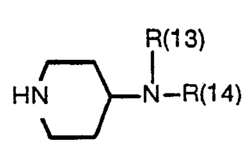

IIIe

in welchen R(6), R(7), R(8), R(9), R(10), R(11), R(12), R(13) und R(14) gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton oder Methylethylketon oder Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160°C, vorzugsweise zwischen 20 und 120°C, umsetzt, oder daß man

b) eine Verbindung der Formel IV

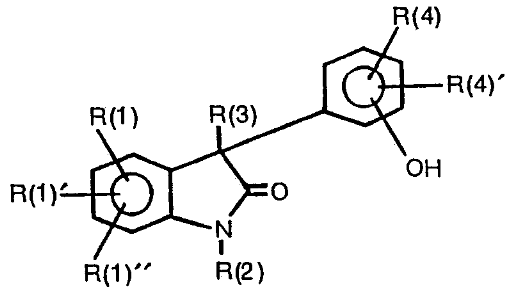

(IV)

in welcher R(1), R(1)', R(1)'', R(2), R(3), R(4) und R(4)' die gleiche Bedeutung wie in Formel I haben, mit einer Verbindung der Formel V,

$$Z—(CH_2)_m—X—(CH_2)_n—R(5) \qquad V$$

in welcher Z gleich wie Y in Formel II definiert ist und in welcher R(5), X, m und n die gleiche Bedeutung wie in Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur zwischen −40 und +60°C, vorzugsweise zwischen −10 und −30°C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin wie beispeilsweise Triethylamin, N-Ethylmorpholin, N-Methyldiisopropylamin oder Pyridin, bei einer Temperatur zwischen 0 und 160°C, vorzugsweise zwischen 20 und 120°C, umsetzt, oder daß man

c) eine Verbindung der Formel VI

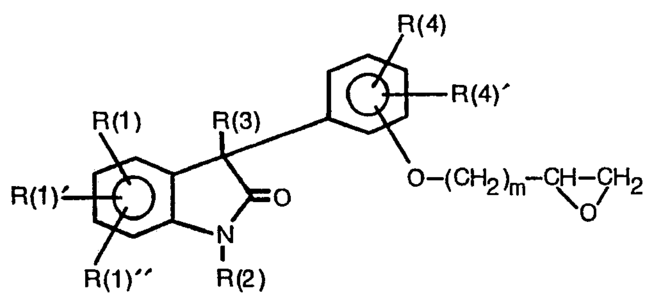

(VI)

in welcher R(1), R(1)', R(1)'', R(2), R(3), R(4), R(4)' und m die gleiche Bedeutung wie in Formel I haben, mit Aminen der Formel IIIa—IIIe ohne Lösungsmittel oder in Gegenwart eines vorzugsweise polaren Lösungsmittels wie Methanol, Isopropanol, Aceton, THF oder Dimethylformamid umsetzt, wobei Verbindungen der Formel I entstehen, worin X = CHOH und n = 1 bedeutet oder daß man

d) eine Verbindung der Formel VII

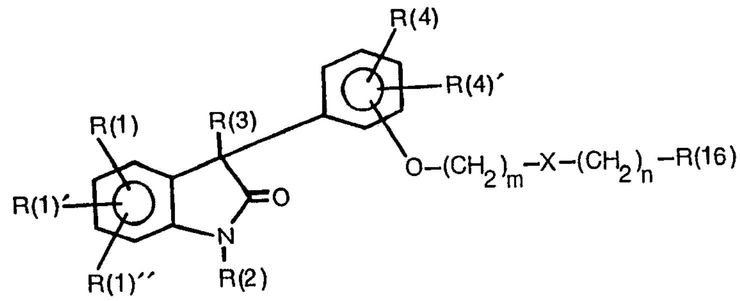

(VII)

in welcher R(1), R(1)', R(1)'', R(2), R(3), R(4), R(4)', X, m und n die gleiche Bedeutung wie in Formel I haben und in welcher R(16) eine der folgenden Gruppen

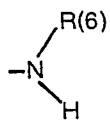

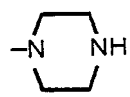
,
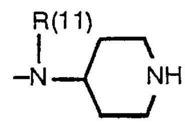
,

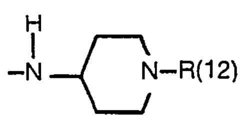
,
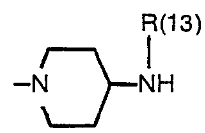

bedeutet, worin R(6), R(11), R(12) und R(13) die gleiche Bedeutung wie in Formel I haben, mit Alkylierungs- oder Acylierungsmitteln nach literaturbekannten Methoden umsetzt, wobei Verbindungen der Formel I entstehen.

Verbindungen der Formel II, die ebenfalls neu und Gegenstand der Erfindung sind, erhält man aus substituierten Anilinen der Formel VIII,

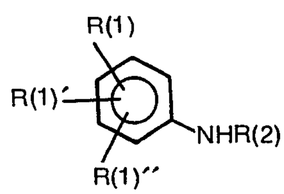

(VIII)

in welcher R(1), R(1)', R(1)'' und R(2) die gleiche Bedeutung wie in Formel I haben, in welcher Formel VIII mindestens eine Orthostellung zur Aminogruppe frei sein muß, durch Umsetzung mit einer Verbindung der Formel IX

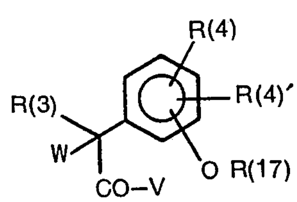

(IX)

in welcher R(3), R(4) und R(4)' die gleiche Bedeutung wie in Formel I haben, R(17) eine unter milden Bedingungen abspaltbare Schutzgruppe wie beispielsweise eine Methyl-, Benzyl- oder Acetylgruppe darstellt, V OH, Cl oder $O(C_1—C_4)$-Alkyl und W OH, OAc, Chlor oder Brom bedeuten, nach den allgemein bekannten Methoden für die Synthese von Amiden aus Aminen und Carbonsäurederivaten, wobei Verbindungen der Formel X

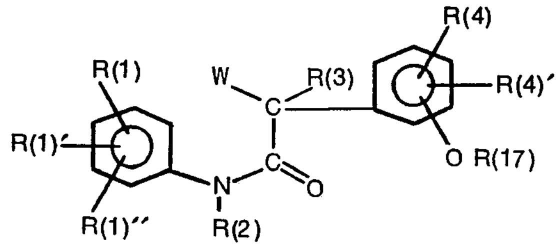

(X)

gebildet werden, in welcher R(1), R(1)', R(1)'', R(2), R(3), R(4) und R(4)' die gleiche Bedeutung wie in Formel I, R(17) und W die gleiche Bedeutung wie in Formel IX besitzen. Sofern W in Formel X eine O-Acetylgruppe bedeutet, ist es zweckmäßig, diese vor der folgenden Cyclisierungsreaktion in eine freie Hydroxylgruppe zu verwandeln. Dies kann auf bekannte Weise durch alkalische Verseifung, z.B. mit Basen wie Ammoniak, Kaliumhydroxyd oder Natriumcarbonat in einem Lösungsmittel wie z.B. Methanol, THF oder Wasser erfolgen.

Anschließende Umsetzung der Verbindungen der Formel X mit wasserabspaltenden Mitteln wie z.B. Polyphosphorsäure oder konzentrierter Schwefelsäure oder mit Lewis-Säure-Katalysatoren wie Aluminiumchlorid, Titantetrachlorid, Zinntetrachlorid, $BF_3$-Etherat ergibt Verbindungen der Formel XI

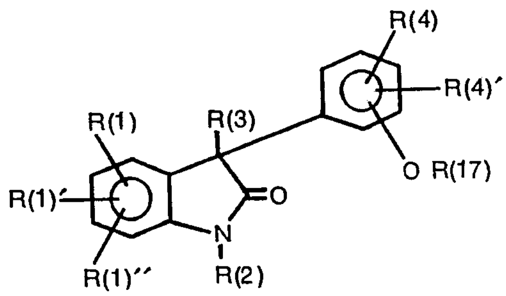

(XI)

worin

R(1), R(1)', R(1)'', R(2), R(3), R(4) und R(4)' dieselbe Bedeutung haben wie in Formel I und R(17) dieselbe Bedeutung hat wie in Formel IX.

Sofern R(3) in Formel XI Wasserstoff bedeutet, ist es zweckmäßig, die Reste R(3), die nicht gleich H sind, durch Alkylierung mit einer Verbindung der Formel XII

$$R(3)\text{—}Y \qquad \text{XII}$$

worin

R(3) die gleiche Bedeutung hat wie in Formel I (jedoch R(3) nicht gleich H) und Y in Formel II definiert ist, zweckmäßig in Gegenwart einer Base wie z.B. Natriumhydrid, Natriumethylat oder einem Lithiumamid, einzuführen, wobei Verbindungen der Formel XI mit R(3) ungleich H gebildet werden.

Ebenso lassen sich in Verbindungen der Formel XI, in der R(2) Wasserstoff bedeutet, Reste R(2), die nicht Wasserstoff bedeuten, durch Alkylierung einführen, z.B. mit einem R(2)-halogenid oder R(2)-sulfonat in Gegenwart einer Base wie z.B. Natriumhydrid oder Kaliumcarbonat mit einem Phasentransferkatalysator, wobei Halogenid Chlorid, Bromid oder Jodid bedeutet.

Aus Verbindungen der Formel XI erhält man durch Abspaltung der Schutzgruppe R(17) unter geeigneten Bedingungen, so beispielsweise durch katalytische Hydrierung für die Benzylgruppe, Umsetzung mit Bortribromid, Trimethyljodsilan oder Pyridinhydrochlorid für die Methylgruppe, oder Kaliumcarbonat in alkoholischer Lösung für die Acetylgruppe, die Verbindungen der Formel IV.

Aus Verbindungen der formel IV lassen sich durch Umsetzung mit Verbindungen der Formel XIII

$$Q\text{—}(CH_2)_m\text{—}X\text{—}(CH_2)_n\text{—}Y \qquad \text{XIII}$$

in welcher X, m und n die gleiche Bedeutung wie in Formel I, Y die gleiche Bedeutung wie in Formel II Q gleich definiert ist wie Y in Formel II in Gegenwart von Basen wie z.B. Natriumhydroxyd, Kaliumcarbonat, Natriummethylat oder Kaliumtertiärbutylat in einem Lösungsmittel wie z.B. Tetrahydrofuran, Methanol, Dimethoxyethan, Aceton, Methylethylketon, Dimethylformamid oder Dimethylsulfoxid Verbindungen der Formel II herstellen.

Verbindungen der Formel VI erhält man aus Verbindungen der Formel IV z.B. mit Epichlorhydrin und Base (für m = 1) nach bekannten Methoden, oder durch Alkylierung von Verbindungen der Formel IV mit Verbindungen der Formel XIV

$$Y—(CH_2)_m—CH = CH_2 \qquad XIV$$

worin
m die gleiche Bedeutung hat wie in Formel I und
Y gleich definiert ist wie in Formel II, wobei Verbindungen der Formel XV

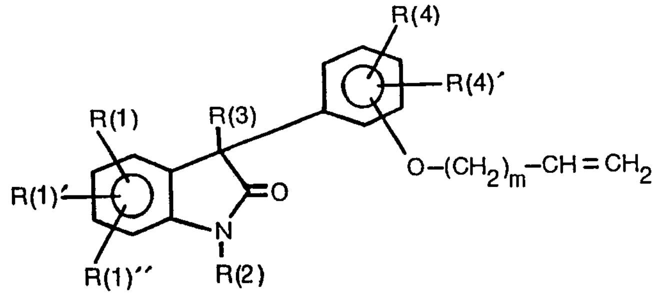
(XV)

gebildet werden, worin R(1), R(1)', R(1)'', R(2), R(3), R(4), R(4)' und m die gleiche Bedeutung wie in Formel I besitzen. Anschließende Epoxidation der Verbindungen XV nach bekannten Methoden, z.B. mit m-Chlorperbenzoesäure in Methylenchlorid ergibt Verbindungen der Formel VI.

Die erfindungsgemäßen Verbindungen der Formel I weisen blutdrucksenkende insbesondere calciumantagonistische Wirkungen auf und können daher zur Behandlung aller Krankheitszustände, die auf einer Störung in dem Calciumhaushalt eines Warmblüters beruhen, verwendet werden.

Ihre calciumantagonistische Wirksamkeit kann an dem biochemischen Testmodell der Verdrängung von tritiummarkiertem Nitrendipin gezeigt werden.

Hierbei werden Membranpräparationen, die isolierte Calciumkanäle enthalten, mit der markierten Substanz beladen. Nach Inkubation mit der Testsubstanz wird die freigesetzte Radioaktivität in der überstehenden Lösung bestimmt. In diesem Modell weisen die erfindungsgemäßen Verbindungen der Formel I $IC_{50}$-Werte von $10^{-6}$ molar bis $10^{-9}$ molar auf. In weiteren Testmodellen, mit denen calciumantagonistische Wirkung nachgewiesen werden kann, z.B. an der Coronardurchströmung am isolierten Meerschweinchenherzen oder am Aktionspotential des isolierten Meerschweinchenpapillarmuskels sind die Verbindungen der Formel I ebenfalls stark wirksam.

Die erfindungsgemaßen Verbindungen der Formel I und ihrer pharmakologisch vertraglichen Salze vermindern das Einströmen von Calcium-Ionen in Zellen und eignen sich daher zu Behandlung des Herz-Kreislaufsystems bei entsprechenden Beschwerden z.B. bei verschiedenen Formen der Angina pectoris, Tachycardie, Herzrhythmusstörungen und Bluthochdruck. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Säugers. Bei oraler Dosierung werden befriedigende Ergebnisse mit Dosen von ab 0,01 mg, vorzugsweise ab 0,1 mg und bis zu 100 mg, vorzugsweise bis zu 20 mg einer Verbindung der Formel I pro kg Körpergewicht erreicht. Beim Menschen variiert die tägliche Dosis zwischen 10 und 800 mg, vorzugsweise 20 bis 500 mg, wobei Einzeldosen von 5 bis 200 mg, vorzugsweise ein bis dreimal täglich, gegeben werden können.

Für intravenöse und intramuskuläre Anwendung beträgt die Dosis 1 bis 300 mg, vorzugsweise 5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-Granulier- und Dragierverfahren, hergestellt und enthalten 0,1% bis etwa 75%, bevorzugt etwa 1% bis etwa 50% des Wirkstoffs.

Die im Anschluß folgenden Beispiele sollen die Erfindung erläutern.

Beispiel 1

3-Benzyl-3-[2-(4-(N-(2-(3,4-dimethoxyphenyl)-ethyl)-N-methylamino)-butoxy)-phenyl]-1-methyl-indolin-2-on-hydrochlorid

a) 2-Methoxy-O-acetylmandelsäure-N-methylanilid

22,4 g (0,1 Mol) 2-Methoxy-O-acetylmandelsäure wurden mit 100 ml Toluol und 28,8 ml (0,4 Mol) Thionylchlorid in Gegenwart von 2 Tropfen DMF 2,5 Stunden unter Rückfluß erhitzt. Das überschüssige Thionylchlorid und das Lösungsmittel wurden i. V. abdestilliert. Der Rückstand wurde in 25 ml Methylenchlorid gelöst. Zu dieser Lösung des Säurechlorids tropfte man 10,7 g N-Methylanilin (0,1 Mol) und 13,8 ml (0,1 Mol) Triethylamin in 100 ml Methylenchlorid innerhalb von 30 Minuten. Man läßt über Nacht stehen und gibt 100 ml Wasser zu dem Reaktionsgemisch. Man trennt die Phasen und wäscht die organische nacheinander mit je 50 ml 1 N HCl, gesättigter $NaHCO_3$-Lösung und Wasser, Nach dem Trocknen mit wasserfreiem Magnesiumsulfat wird das Lösungsmittel i. V. verdampft und der Rückstand mit Diisopropylether verrieben und abgesaugt. Man erhält 30,1 g Kristalle vom Fp. 84—86°C. H-NMR ($CDCl_3$): δ = 6,4—7,5 (m, 9H), 6,23 (s, 1H), 3,27 (s, 3H), 3,2 (s, 3H), 2,08 (s, 3H).

b) 2-Methoxymandelsäure-N-methylanilid

19,5 g der oben erhaltenen O-Acetylverbindung werden in 100 ml methanolischem Ammoniak 12 Stunden bei Zimmertemperatur gerührt. Nach Abdampfen des Lösungsmittels erhält man ein Öl, das beim Stehen durchkristallisiert. Ausbeute 14,9 g.

Nach Umkristallisieren aus Isopropanol schmilzt das Produkt bei 93—94°.

| | | | |
|---|---|---|---|
| Ber ($C_{16}H_{17}NO_3$) | C 70,8 | H 6,3 | N 5,2 |
| Gef | C 71,0 | H 6,2 | N 5,1 |

c) 1-Methyl-3-(2-methoxyphenyl)-indolin-2-on

32,2 g (0,12 Mol) 2-Methoxymandelsäure-N-methylanilid werden bei 50°C in 325 g Polyphosphorsäure eingetragen. Die Mischung wird unter Rühren 3 Stunden auf 110—120° erhitzt und dann auf 1,5 l Eiswasser gegeben. Man extrahiert zweimal mit je 500 ml Methylenchlorid, wäscht die organischen Phasen zweimal mit Wasser, trocknet und engt i. Vak. ein. Der ölige Rückstand kristallisiert beim Verreiben mit Cyclohexan. Ausbeute 25.2 g Fp 100—102°C.

$^1$H-NMR ($CDCl_3$): δ = 7,4—6,6 (m, 8H), 4,8 (s, 1H), 3,65 (s, 3H), 3,23 (s, 3H)

d) 3-Benzyl-1-methyl-3-(2-methoxyphenyl)-indolin-2-on

20,24 g (0,08 Mol) 1-Methyl-3-(2-methoxyphenyl)-indolin-2-on werden in 200 ml trockenem THF gelöst. Dann werden 9.2 g (0,08 Mol) Kaliumtertiärbutylat zugegeben und 15 Minuten bei Zimmertemperatur gerührt. Bei 10° wird eine Lösung von 12,1 ml Benzylbromid in 20 ml trockenem THF zugetropft und 3 Stunden bei Zimmertemperatur nachgerührt. Anschließend wird i. Vak. eingeengt, der Rückstand zwischen Wasser und Essigester verteilt, die organische Phase zweimal mit Wasser gewaschen, getrocknet und eingeengt. Man erhält 26,1 g Produkt vom Fp. 174—176°C.

$^1$H-NMR ($CDCl_3$): δ = 6,2—7,4 (m, 12H), 7,5—7,8 (m, 1H), 3,5 (s, 2H), 3,33 (s, 3H), 2,75 (s, 3H)

e) 3-Benzyl-3-(2-hydroxyphenyl)-1-methyl-indolin-2-on

25 g (0,073 Mol) 3-Benzyl-1-methyl-3-(2-methoxyphenyl)-indolin-2-on werden in 250 ml Methylenchlorid gelöst. Man kühlt auf 0—5° ab und tropft während 30 Minuten eine Lösung von 7 ml (0,074 Mol) Bortribromid in 50 ml Methylenchlorid zum, rührt 1 Stunde bei Zimmertemperatur nach und gießt auf Eiswasser. Die organische Phase wird abgetrennt, zweimal mit Wasser gewaschen, über Magnesiumsulfat getrocknet und eingeengt. Man erhält 17,8 g kristallines Produkt vom Fp. 188—190°C.

$^1$H-NMR ($CDCl_3$): δ = 6,4—7,3 (m, 13H), 3,62 (AB, 2H), 2,9 (s, 3H)

f) 3-Benzyl-3-[2-(4-brombutoxy)-phenyl]-1-methyl-inodolin-2-on

16,5 g (0,05 Mol) 3-Benzyl-3-(2-hydroxyphenyl)-1-methyl-indolin-2-on werden in 250 ml 2-Butanon mit 20,7 g (0,15 Mol) gemahlenem, wasserfreiem Kaliumcarbonat und 17,2 ml (0,15 Mol) 1,4-Dibrombutan 5 Stunden unter Rückfluß gerührt. Man saugt die heiße Mischung ab und engt das Filtrat i. Vak. ein. Der kristalline Rückstand wird mit Petrolether verrieben und das Produkt nach Absaugen des Lösungsmittels isoliert. Man erhält 19,3 g Produkt vom Fp. 174—5°C.

$^1$H-NMR ($CDCl_3$): δ = 6,2—7,3 (m, 12H), 7,5—7,8 (m, 1H), 2,7 (s, 3H), 1,2—4,6 (m, 4H)

g) 3-Benzyl-3-[2-(4-(N-(2-(3,4-dimethoxyphenyl)-ethyl)-N-methylamino)-butoxy)-phenyl]-1-methyl-indolin-2-on-hydrochlorid

5,56 g (0,012 Mol) der Brombutylverbindung werden in 30 ml DMF mit 1,66 g (0,012 Mol) gemahlenem Kaliumcarbonat und 3,51 g (0,018 Mol) N-Methylhomoveratrylamin 6 Stunden auf 80—90°C erwärmt. Man gießt auf 250 ml Wasser und saugt den Niederschlag ab. Das Rohprodukt wird durch Chromatographie an

250 g Kieselgel mit Methylenchlorid/Methanol 9:1 als Laufmittel gereinigt. Das gereinigte Produkt wird mit etherischer HCl in das Hydrochlorid überführt. Fp. 135—137°C.

Ber ($C_{37}H_{43}ClN_2O_4$) C 72,2 H 7,0 N 4,6 Cl 5,8
Gef C 71,9 H 7,1 N 4,2 Cl 5,6

$^1$H-NMR (Base in $CDCl_3$): δ = 6,2—7,4 (m, 15H), 7,5—7,8 (m, 1H), 3,0—4,0 (m, 4H), 3,83 (s, 6H), 2,73 (s, 3H), 2,40 (s, 3H), 1,0—1,5 (m, 4H)

$IC_{50}$-Wert in Nitrendipin-Verdrangungstest: $8 \times 10^{-6}$

Beispiel 2

3-Benzyl-1-methyl-3-[2-(4-(4-(2-(3,4,5-trimethoxyphenyl)-ethyl)-piperazinyl)-butoxy)-phenyl]-indolin-2-on-dihydrochlorid

Versuch analog Beispiel 1g) aus 3-Benzyl-3-[2-(4-brombutoxy)-phenyl]-1-methylindolin-2-on und [2-(3,4,5-Trimethoxyphenyl)-ethyl]-piperazin.

Das Dihydrochlorid schmilzt bei 165—167°C.

Ber ($C_{41}H_{51}Cl_2N_3O_5$) N 5,7 Cl 9,6
Gef N 5,4 Cl 9,6

$^1$H-NMR (Base in $CDCl_3$): δ = 6,2—7,5 (m, 12H), 7,5—7,85 (m, 1H), 3,1—4,0 (m, 13H), 2,0—3,0 (m, 11H), 2,72 (s, 3H), 1,0—1,5 (m, 4H)

$IC_{50}$-Wert: $1 \times 10^{-6}$

Beispiel 3

3-[2-(4-(N-(2-(3,4-Dimethoxyphenyl)-ethyl)-N-methyl-amino)-butoxy)-phenyl]-3-isopropyl-1-methylindolin-2-on-hydrochlorid

a) 3-Isopropyl-3-(2-methoxyphenyl)-1-methylindolin-2-on

35,4 g (0,14 Mol) 3-(2-Methoxyphenyl)-1-methylindolin-2-on werden in 350 ml trockenem THF mit 16,1 g (0,14 Mol) Kaliumtertiärbutylat während 15 Minuten bei Raumtemperatur gerührt. Dann tropft man bei 10°C eine Lösung von 14,3 ml 2-Jodpropan in 70 ml trockenem THF zu und rührt 4 Stunden bei Raumtemperatur nach. Man läßt über Nacht stehen und verteilt das Reaktionsgemisch zwischen Wasser und Essigester. Nach Eindampfen der organischen Phase erhält man 4,8 g Produkt von Fp. 113—115°C.

$^1$H-NMR ($CDCl_3$): δ = 7,3—7,5 (m, 1H), 6,5—7,3 (m, 7H), 3,42 (s, 3H), 3,2 (s, 3H), 2,9 (m, 1H), 0,92 (dd, 6H)

b) 3-(2-Hydroxyphenyl)-3-isopropyl-1-methylindolin-2-on

Zu 5,9 g (0,02 Mol) 3-Isopropyl-3-(2-methoxyphenyl)-1-methylindolin-2-on in 75 ml Methylenchlorid wird bei 0°C eine Lösung von 1,92 ml (0,02 Mol) Bortribromid in 20 ml Methylenchlorid während 1 Stunde zugetropft. Nach 30 Minuten gießt man auf Eiswasser, wäscht die organische Phase mit Wasser, trocknet und dampft ein. Der Rückstand wird mit kaltem Toluol verrieben und abgesaugt. 4,8 g, Fp. 172—174°C.

$^1$H-NMR ($CDCl_3$): δ = 10,7 (s, 1H), 6,4—7,6 (m, 8H), 3,45 (m, 1H), 3,2 (s, 3H), 0,7 (dd, 6H)

c) 3-[2-(4-Brombutoxy-phenyl]-3-isopropyl-1-methyl-indolin-2-on

19,7 g (0,07 Mol) der Hydroxyphenylverbindung werden in 250 ml 2-Butanon mit 29 g (0,21 Mol) gemahlenem Kaliumcarbonat und 24,8 ml (0,21 Mol) Dibrombutan 4 Stunden unter Rühren zum Sieden erhitzt. Der entstandene Niederschlag wird abgesaugt und das Filtrat i. Vak., zuletzt im Hochvakuum eingedampft. Der Rückstand kristallisiert beim Stehen. Man verrührt mit Petroläther und saugt ab. 23, 8 g, Fp. 98—100°C.

$^1$H-NMR ($CDCl_3$): δ = 6,5—7,6 (m, 8H), 3,25 (s, 3H), 2,5—4,0 (m, 5H), 1,3—1,7 (m, 4H), 0,95 (dd, 6H)

d) 3-[2-(4-(N-(2-(3,4-Dimethoxyphenyl)-ethyl)-N-methylamino)-butoxy)-phenyl]-3-isopropyl-1-methyl-indolin-2-on-hydrochlorid

5,0 (0,012 Mol) des Brombutoxyderivatives werden in 30 ml DMF mit 1,66 g (0,012 Mol) Kaliumcarbonat und 3,51 g (0,018 Mol) N-Methylhomoveratrylamin 5 Stunden auf 80—90°C erwärmt. Man gießt auf Eiswasser und extrahiert dreimal mit Essigester. Die vereinigten organischen Phasen werden mit Wasser zweimal gewaschen, getrocknet und eingeengt. Das Rohprodukt wird durch Chromatographie an 250 g Kieselgel mit Methylenchlorid/Methanol 9:1 als Elutionsmittel gereinigt. Nach Überführung in das Hydrochlorid schmilzt die Verbindung bei 120°C.

Ber ($C_{33}H_{43}Cl\ N_2O_4$) C 69,9 H 7,6 N 4,9 Cl 6,3
Gef C 69,5 H 8,0 N 4,8 Cl 6,5

$^1$H-NMR (Base in $CDCl_3$): δ = 7,4—7,7 (m, 1H), 6,5—7,4 (m, 10H), 3,85 (s, 6H), 2,3 (s, 3H), 0,9 (dd, 6H)

$IC_{50}$-Wert: $2,2 \times 10^{-8}$

Beispiel 4

3-Isopropyl-1-methyl-3-[2-(4-(4-(2-(3,4,5-trimethoxyphenyl)-ethyl)-piperazinyl)-butoxy)-phenyl]-indolin-2-on-dihydrochlorid

Herstellung analog Beispiel 3d) aus 3-[2-(4-Brombutoxy)-phenyl]-3-isopropyl-1-methyl-indolin-2-on und [2-(3,4,5-Trimethoxyphenyl)-äthyl]-piperazin.

Fp. 223—225°C
Ber ($C_{37}H_{51}Cl_2N_3O_5$) N 6,1 Cl 10,3
Gef N 5,9 Cl 10,2
$^1$H-NMR (Base in $CDCl_3$): δ = 7,4—7,65 (m, 1H), 6,2—7,4 (m, 9H), 3,80 und 3,83 (2s, 9H), 3,23 (s, 3H), 1,16—1,6 (m, 4H), 0,9 (dd, 6H)
$IC_{50}$-Wert: 5 × $10^{-9}$

Beispiel 5

3-[2-(4-(4-(4-Bis-(4-fluorphenyl)-butyl)-piperazin-1-yl)-butoxy)-phenyl]-3-isopropyl-1-methyl-indolin-2-on-dihydrochlorid

Herstellung analog Beispiel 3d aus 3-[2-(4-Brombutoxy)-phenyl]-3-isopropyl-1-methylindolin-2-on und [4-Bis-(4-fluorphenyl)-butyl]-piperazin.
Fp. 180—1°C
Ber ($C_{42}H_{51}F_2Cl_2N_3O_2$) C 68,3 H 7,0 N 5,7
Gef C 67,9 H 7,0 N 5,4
$IC_{50}$-Wert: 1,3 × $10^{-8}$

Beispiel 6

3-Isopropyl-1-methyl-3-[2-(4-(4-(3,4,5-trimethoxybenzyl)-piperazin-1-yl)-butoxy)-phenyl)-indolin-2-on-dihydrochlorid

Herstellung analog Beispiel 3d aus 3-[2-(4-Brombutoxy)-phenyl]-3-isopropyl-1-methylindolin-2-on und 3,4,5-Trimethoxybenzylpiperazin.
Fp. 235—7°C
Ber ($C_{37}H_{49}Cl_2N_3O_5$) C 64,7 H 7,2 N 6,1 Cl 10,3
Gef C 64,5 H 7,2 N 6,2 Cl 10,5
$IC_{50}$-Wert: 4,4 × $10^{-8}$

Beispiel 7

3-Isopropyl-1-methyl-3-[4-(4-(3,4,5-trimethoxyphenylacetyl)-piperazin-1-yl)-butoxyphenyl]-indolin-2-on-hydrochlorid

Herstellung analog Beispiel 3d aus 3-[2-(4-Brombutoxy)-phenyl]-3-isopropyl-1-methyl-indolin-2-on und 3,4,5-Trimethoxyphenylacetylpiperazin.
Fp. 140°C (Zers).
Ber ($C_{37}H_{48}ClN_3O_6$) C 66,7 H 7,3 N 6,3 Cl 5,3
Gef C 66,9 H 7,3 N 6,0 Cl 5,4
$IC_{50}$-Wert: 4,2 × $10^{-8}$

Beispiel 8

1,5-Dimethyl-3-isopropyl-3-[4-(4-(2-(3,4,5-trimethoxyphenyl)-äthyl)-piperazinyl)-butoxy)-phenyl]-indolin-2-on-dihydrochlorid

Herstellung analog Beispiel 3d aus 3-[2-(4-Brombutoxy)-phenyl]-1,5-dimethyl-3-isopropylindolin-2-on und [2-(3,4,5-Trimethoxyphenyl)-ethyl]-piperazin.
Fp. 242°C.
Ber ($C_{38}H_{53}Cl_2N_3O_5$) C 64,9 H 7,6 N 6,0 Cl 10,1
Gef C 64,6 H 7,4 N 6,1 Cl 9,9

$^3$H-Nitrendipin-Bindungstest

Die Prüfung der Wirksamkeit von Substanzen im $^3$H-Nitrendipin-Bindungstest führten wir in einer aus dem Cortex des Rattenhirns gewonnenen und mehrfach gewaschenen Membranpräparation aus, wobei wir im wesentlichen die von R. J. Gould et al. (Proc. Natl. Acad. Sci. USA *79*, 3656 [1982]) beschriebene Methode anwendeten. Die auf 1:1500 mit TRIS-Puffer pH 7,4 (50 mM TRIS-HCl, 150 mM NaCl, 1,0 mM $CaCl_2$ und 0,001 Gew.-%, bezogen auf TRIS-HCl, NaCl und $CaCl_2$ in Lösung, einer neutralen oberflächenaktiven Substanz, wie z.B. Genapol®) verdünnte Membransuspension wurde in 5 ml-Partitionen mit $^3$H-Nitrendipin (0,1 nM im Test, spez. Aktivität 81,3 Ci/m Mol) 60 Min. bei 25°C im Schüttelwasserbad inkubiert. Die Abtrennung der Membranfraktionen wurde durch Vakuumfiltration über Whatman-GF/F-Glasfaserfilter vorgenommen und die Radioaktivität im Flüssigkeitsszintillationszähler gemessen. Die unspezifische $^3$H-Nitrendipin-Bindung bestimmten wir in Gegenwart von 1 µM Nifedipin.

Analog zu den in den aufgeführten Beispielen genannten Verbindungen wurden folgende Substanzen hergestellt:

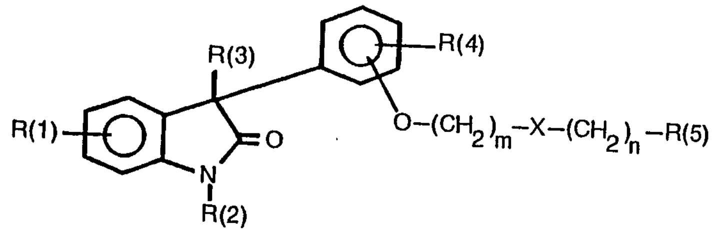

| Bsp. Nr. | $R^1$ | $R^2$ | $R^3$ | $R^4$ | Ether-seitenk. in Pos. | m | X | n | $R^5$ | NMR |
|---|---|---|---|---|---|---|---|---|---|---|
| 9 | H | $CH_3$ | i-$C_3H_7$ | H | 2′ | 3 | $CH_2$ | 0 | 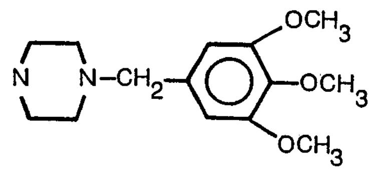 | 7,4—7,6 (m, 1H), 6,5—7,4 (m, 7H), 6,5 (s, 2H), 3,2 (s, 3H), 1,3—1,7 (m, 4H), 0,9 (dd, 6H) |
| 10 | H | $CH_3$ | n-$C_6H_{13}$ | H | 2′ | 3 | $CH_2$ | 0 | 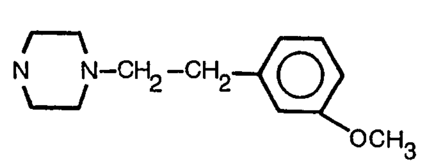 | 7,4—7,65 (m, 1H), 6,4—7,4 (m, 11H), 3,8 (s, 3H), 3,3 (s, 3H), 1,0—1,7 (m, 12H), 0,85 (m, 3H) |
| 11 | H | $CH_3$ | n-$C_{12}H_{25}$ | H | 2′ | 3 | $CH_2$ | 0 | 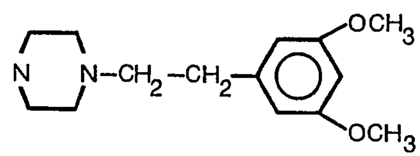 | 6,3—7,7 (m, 11H), 3,5—3,9 (s, 6H + m, 2H), 1,0—1,8 (m, 24H), 0,87 (m, 3H) |
| 12 | H | $CH_3$ | $CH_2$—CH=$CH_2$ | H | 2′ | 3 | $CH_2$ | 0 | 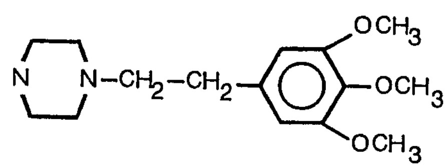 | 6,5—7,7 (m, 8H), 6,45 (s, 2H), 3,8 + 3,85 (2s, 9), 5,0—5,9 (m, 3H), 3,2—3,7 (m + s, 7H), 2,0—3,1 (m, 14H), 1,3—1,7 (m, 4H) |
| 13 | H | $CH_3$ | i-$C_3H_7$ | H | 2′ | 3 | $CH_2$ | 0 | 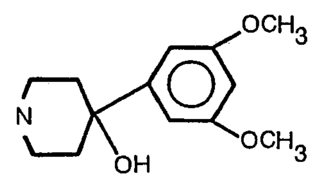 | 7,4—7,65 (m, 1H), 6,4—7,4 (m, 10H), 3,84 (s, 6H), 3,3 (s, 3H), 0,9 (dd, 6H) |

| Bsp. Nr. | R¹ | R² | R³ | R⁴ | Ether-seitenk. in Pos. | m | X | n | R⁵ | NMR |
|---|---|---|---|---|---|---|---|---|---|---|
| 14 | H | $CH_3$ | 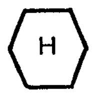 | H | 2′ | 3 | $CH_2$ | 0 | 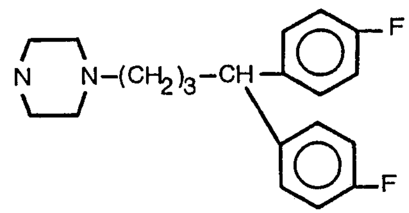 | 7,4—7,7 (m, 1H), 6,5—7,4 (m, 15H), 3,7 (t, 3H), 2,3—3,0 (m, 13H), 1,1—1,7 (m, 18H) |
| 15 | H | $C_2H_5$ | 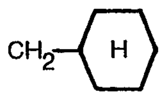 | H | 2′ | 3 | $CH_2$ | 0 | 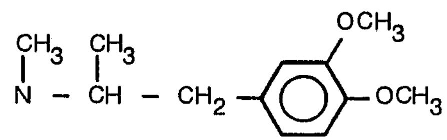 | 6,5—7,65 (m, 11H), 3,84 (s, 6H), 2,35 (s, 3H), 0,85—1,8 (m, 21H) |
| 16 | H | $C_3H_7$ | 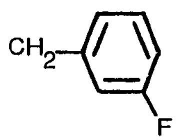 | H | 2′ | 3 | $CH_2$ | 0 | 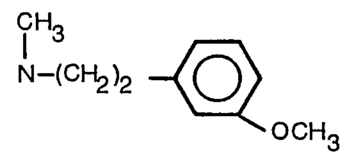 | 6,5—7,7 (m, 16H), 3,8 (s, 3H), 3,3—3,9 (m, 6H), 2,35 (s, 3H), 0,95 (t, 3H) |
| 17 | H | $CH_3$ | $i\text{-}C_3H_7$ | H | 2′ | 3 | $CH_2$ | 0 | 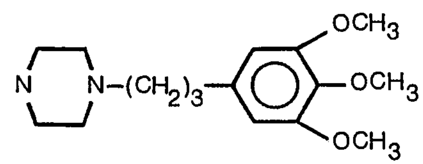 | 7,4—7,65 (m, 1H), 6,5—7,4 (m, 7H), 6,4 (s, 2H), 3,8—3,9 (2s, 9H), 1,2—1,7 (m, 6H), 0,9 (dd, 6H) |
| 18 | H | 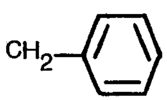 | $C_2H_5$ | H | 2′ | 3 | $CH_2$ | 0 | 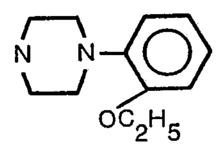 | 6,5—7,7 (m, 17H), 4,0 (q, 2H), 3,65 (t, 2H), 2,3—3,5 (m, 12H), 1,42 (t, 3H), 0,9 (t, 3H) |
| 19 | H | $CH_3$ | $i\text{-}C_3H_7$ | H | 2′ | 4 | $CH_2$ | 0 | 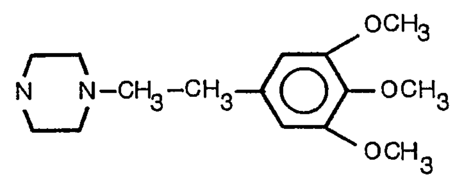 | 7,4—7,65 (m, 1H), 6,5—7,4 (m, 7H), 6,4 (s, 2H), 3,8 + 3,85 (2S, 9H), 3,3 (s, 3H), 1,1—1,6 (m, 6H), 0,9 (dd, 6H) |

| Bsp. Nr. | R[1] | R[2] | R[3] | R[4] | Ether-seitenk. in Pos. | m | X | n | R[5] | NMR |
|---|---|---|---|---|---|---|---|---|---|---|
| 20 | H | $CH_3$ | i-$C_3H_7$ | H | 2′ | 3 | $CH_2$ | 0 | 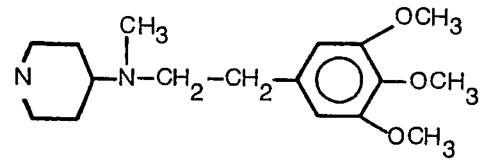 | 6,5—7,65 (m, 8H), 6,4 (s, 2H), 3,8 + 3,85 (2s, 9H), 3,3 (s, 3H), 2,3 (s, 3H), 0,95 (dd, 6H) |
| 21 | H | $CH_3$ | n-$C_3H_7$ | H | 2′ | 3 | $CH_2$ | 0 | 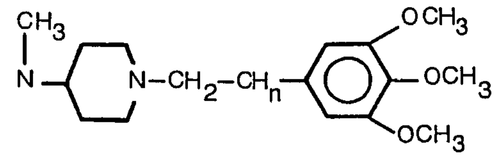 | 6,5—7,7 (m, 8H), 6,4 (s, 2H), 3,8 + 3,83 (2s, 3H), 0,9 (t, 3H) |
| 22 | H | $CH_3$ | i-$C_3H_7$ | H | 3′ | 2 | $CH_2$ | 0 | 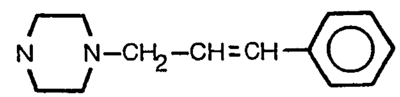 | 7,4—7,6 (m, 1H), 6,0—7,4 (m, 14H), 3,7 (m, 2H), 3,2 (s, 3H), 3,1—3,4 (m, 2H), 0,95 (dd, 6H) |
| 23 | 5-Cl | $CH_3$ | i-$C_3H_7$ | H | 3′ | 2 | $CH_2$ | 0 | 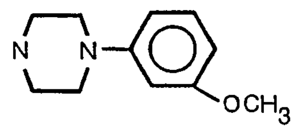 | 6,4—7,6 (m, 12H), 3,7 (s, 3H), 2,3—3,8 (m, 13H), 3,3 (s, 3H), 0,9 (dd, 6H) |
| 24 | 7-$CH_3$ | $CH_3$ | 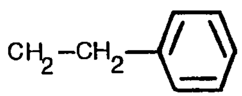 | H | 2′ | 3 | $CH_2$ | 0 | 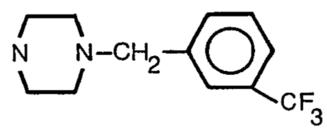 | 6,5—7,4 (m, 16H), 2,2—3,8 (m, 24H), 1,3—1,7 (m, 4H) |
| 25 | H | $CH_3$ | n-$C_3H_7$ | H | 2′ | 3 | $CH_2$ | 0 | 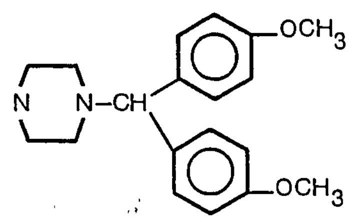 | 6,4—7,6 (m, 16H), 3,8 (s, 6H), 3,2 (s, 3H), 2,2—3,8 (m, 14H), 1,3—1,7 (m, 6H), 0,9 (t, 3H) |
| 26 | H | $CH_3$ | i-$C_3H_7$ | 3′-$OCH_3$ | 2′ | 3 | $CH_2$ | 0 | 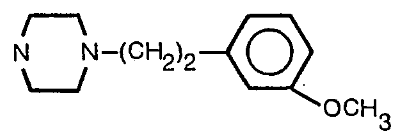 | 7,4—7,7 (m, 1H), 6,4—7,4 (m, 10H), 3,8 + 3,85 (2s, 6H), 2,2—3,2 (m, 15H), 0,9 (dd, 6H) |

| Bsp. Nr. | $R^1$ | $R^2$ | $R^3$ | $R^4$ | Ether-seitenk. in Pos. | m | X | n | $R^5$ | NMR |
|---|---|---|---|---|---|---|---|---|---|---|
| 27 | H | $CH_3$ | $i\text{-}C_3H_7$ | $3'\text{-}OCH_3$ | 2' | 3 | $CH_2$ | 0 | 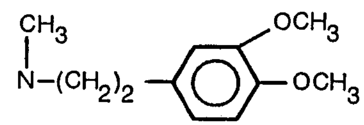 | 6,4—7,7 (m, 10H), 3,80—3,85 (3s, 9H), 3,3 (s, 3H), 2,3 (s, 3H), 0,9 (dd, 6H) |
| 28 | H | $CH_3$ | $C_2H_5$ | H | 4' | 1 | $CH_2$ | 0 | 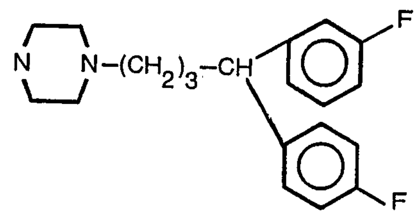 | 7,4—7,65 (m, 1H), 6,4—7,4 (m, 15H), 3,4—4,1 (m, 3H), 3,4 (s, 3H), 1,8—3,0 (m, 16H), 1,1 (t, 3H) |
| 29 | H | $CH_3$ | 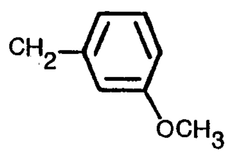 | $CH_3$ | 4' | 2 | $CH_2$ | 0 | 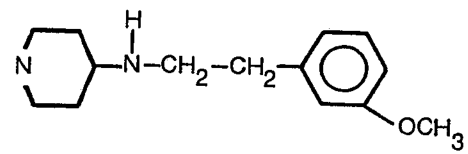 | 6,4—7,7 (m, 16H), 3,8 + 3,85 (2s, 6H), 3,2 (s, 3H), 2,25 (s, 3H) |
| 30 | H | $CH_3$ | 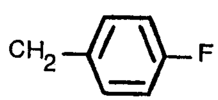 | H | 2' | 3 | $CH_2$ | 0 | 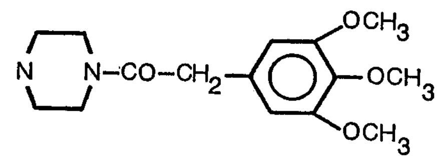 | 7,4—7,6 (m, 1H), 6,5—7,4 (m, 11H), 6,4 (s, 2H), 3,8 und 3,85 (2s, 9H), 3,2 (s, 2H), 2,6—3,8 (m, 13H) |
| 31 | H | $CH_3$ | $i\text{-}C_3H_7$ | H | 2' | 3 | $CH_2$ | 0 | 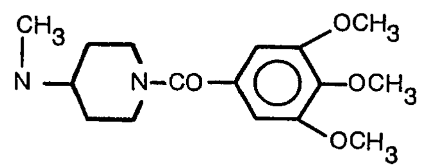 | 7,4—7,7 (m, 1H), 6,5—7,4 (m, 7H), 6,4 (s, 2H), 3,8 (s, 9H), 3,7 (m, 2H), 3,2 (s, 3H), 2,3 (s, 3H), 0,9 (dd, 6H) |
| 32 | H | $CH_3$ | $i\text{-}C_3H_7$ | H | 2' | 3 | $CH_2$ | 0 | 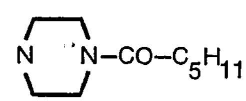 | 7,4—7,6 (m, 1H), 6,5—7,4 (m, 7H), 3,2 (s, 3H), 0,9 (dd, 6H), 0,85 (t, 3H) |

| Bsp. Nr. | $R^1$ | $R^2$ | $R^3$ | $R^4$ | Ether-seitenk. in Pos. | m | X | n | $R^5$ | NMR |
|---|---|---|---|---|---|---|---|---|---|---|
| 33 | H | $CH_3$ | i-$C_3H_7$ | H | 2′ | 3 | $CH_2$ | 0 | 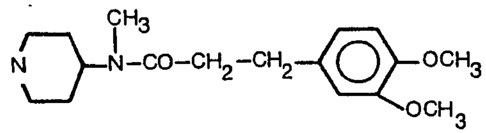 | 7,4—7,6 (m, 1H), 6,5—7,4 (m, 10H), 3,8 (s, 6H), 3,2 (s, 3H), 2,8 (s, 3H), 0,9 (dd, 6H) |
| 34 | H | $CH_3$ | $C_2H_5$ | H | 2′ | 3 | C=O | 0 | 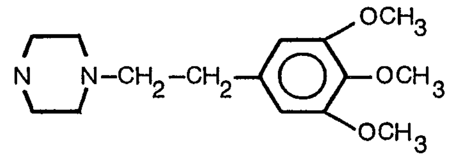 | 6,5—7,7 (m, 8H), 6,4 (s, 2H), 3,80, 3,83 (2s, 9H), 3,2 (s, 3H), 1,1 (t, 3H) |
| 35 | H | $CH_3$ | i-$C_3H_7$ | H | 2′ | 2 | 0 | 2 | 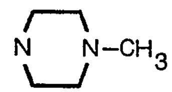 | 7,4—7,6 (m, 1H), 6,5—7,4 (m, 7H), 3,5—4,1 (m, 6H), 3,2 (s, 3H), 2,4 (s, 3H), 1,0 (dd, 6H) |
| 36 | H | $CH_3$ | n-$C_6H_{13}$ | H | 2′ | 1 | CH(OH) | 1 | 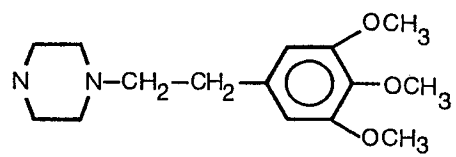 | 7,4—7,7 (m, 1H), 6,5—7,4 (m, 7H), 6,4 (s, 2H), 3,8 and 3,85 (2s, 9H), 3,2 (s, 3H), 0,85 (t, 3H) |
| 37 | H | $CH_3$ | i-$C_3H_7$ | H | 2′ | 2 | S | 2 | 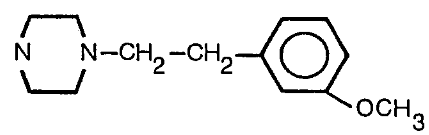 | 6,4—7,7 (m, 12H), 3,8 (s, 3H), 3,2 (s, 3H), 2,3—4,0 (m, 21H), 0,95 (dd, 6H) |

**Patentansprüche für die Vertragsstaaten: BE CH DE FR GB IT LI LU NL SE**

1. Verbindung der Formel I

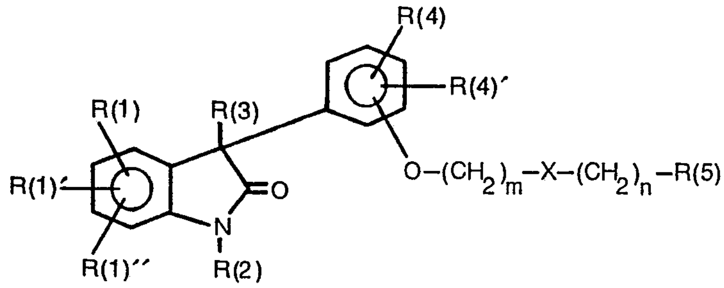

(I)

in welcher

R(1), R(1)' und R(1)'' gleich oder verschieden und voneinander unabhängig sind und Wasserstoff, $(C_1—C_4)$-Alkyl, $(C_1—C_3)$-Alkoxy, F, Cl, Br, $CF_3$, Nitro, Hydroxy, Acetamido oder Amino,

R(2) Wasserstoff, $(C_1—C_{10})$-Alkyl, geradkettig oder verzweigt, $(C_3—C_{10})$-Alkenyl, geradkettig oder verzweigt, Phenyl-$(C_1—C_4)$-alkyl, wobei der Phenylring unsubstituiert oder durch einen, zwei oder drei Substituenten der Gruppe $(C_1—C_4)$-Alkyl, $(C_1—C_3)$-Alkoxy, F, Cl, $CF_3$, $(C_1—C_2)$-Alkylendioxy oder Nitro substituiert ist,

R(3) Wasserstoff, $(C_1—C_{15})$-Alkyl, geradkettig oder verzweigt, $(C_3—C_{15})$-Alkenyl, geradkettig oder verzweigt, $(C_4—C_8)$-Cycloalkyl, $(C_4—C_8)$-Cycloalkyl-$(C_1—C_4)$alkyl, Phenyl oder Phenyl-$(C_1—C_4)$-alkyl, wobei der Phenylrest unsubstituiert oder durch einen, zwei oder drei Substituenten der Gruppe $(C_1—C_4)$-Alkyl, $(C_1—C_3)$-Alkoxy, F, Cl, $CF_3$, $(C_1—C_2)$-Alkylendioxy oder Nitro substituiert ist

R(4) und R(4)' gleich oder verschieden und voneinander unabhängig Wasserstoff, $(C_1—C_4)$-Alkyl, $(C_1—C_3)$-Alkoxy, F, Cl, $CF_3$,

m 1, 2,

n 0, 1, 2

X eine $CH_2$-Gruppe,

R(5) eine der folgenden Gruppe

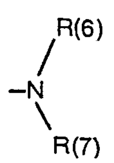

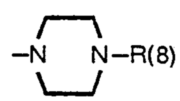

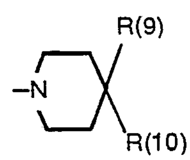

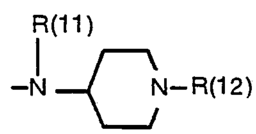

oder

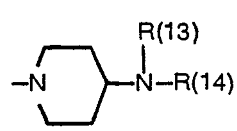

worin

R(6) und R(7) gleich oder verschieden voneinander unabhängig Wasserstoff, $(C_1—C_{10})$-Alkyl, $(C_4—C_8)$-Cycloalkyl, $(C_4—C_8)$-Cycloalkyl-$(C_1—C_4)$-alkyl, Pyridyl-$(C_1—C_4)$-alkyl, Phenyl-$(C_1—C_6)$-alkyl, Benzhydryl oder Benzhydryl-$(C_1—C_4)$-alkyl, wobei die Phenylrest jeweils unsubstituiert oder durch einen, zwei oder drei Reste aus der Gruppe $(C_1—C_4)$-Alkyl, $(C_1—C_4)$-Alkoxy, $(C_1—C_2)$-Alkylendioxy, F, Cl, Br, $CF_3$ oder Hydroxy substituiert sind,

R(8) Wasserstoff, $(C_1—C_{10})$-Alkyl, geradkettig oder verzweigt, $(C_1—C_8)$-Alkanoyl, Pyridyl, Pyrimidinyl, Phenyl, Phenyl-$(C_1—C_4)$-alkyl, Phenyl-$(C_3—C_5)$-alkenyl, Benzhydryl oder Benzhydryl —$(C_1—C_4)$-alkyl, Phenyl-$(C_1—C_4)$-alkanoyl oder Benzoyl, wobei die Phenylreste jeweils unsubstituiert oder durch einen, zwei oder drei Reste aus der Gruppe $(C_1—C_4)$-Alkyl, $(C_1—C_4)$-Alkoxy, $(C_1—C_2)$-Alkylendioxy, F, Cl, Br, $CF_3$ oder Hydroxy substituiert sind,

R(9) Wasserstoff, $(C_1—C_{10})$-Alkyl, Phenyl, Phenyl-$(C_1—C_4)$-alkyl, wobei der Phenylrest jeweils unsubstituiert oder durch einen, zwei oder drei Reste aus der Gruppe $(C_1—C_4)$-Alkyl, $(C_1—C_4)$ Alkoxy, $(C_1—C_2)$-Alkylendioxy, F, Cl, Br, $CF_3$ oder Hydroxy substituiert ist,

R(10) Wasserstoff, Hydroxy, oder $(C_1—C_4)$-Alkoxy, und

R(11) und R(12) bzw, R(13) und R(14) gleich oder verschieden und voneinander unabhängig Wasserstoff, $(C_1—C_{10})$ alkyl, geradkettig oder verzweigt, $(C_1—C_6)$-Alkanoyl, Phenyl-$(C_1—C_4)$-alkyl, Benzhydryl oder Benzhydryl-$(C_1—C_4)$-alkyl, Phenyl-$(C_1—C_4)$-alkanoyl, oder Benzoyl, wobei die Phenylreste jeweils unsubstituiert oder durch einen, zwei oder drei Rest aus der Gruppe $(C_1—C_4)$-Alkyl, $(C_1—C_4)$-Alkoxy, $(C_1—C_2)$-Alkylendioxy, F, Cl, Br, $CF_3$ oder Hydroxy substituiert sind, bedeuten, sowie die Salze der Verbindungen der Formel I mit pharmazeutisch akzeptablen Säuren.

2. Verbindung nach Anspruch 1, dadurch gekennzeichnet, daß

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)'' Wasserstoff,

R(2) Wasserstoff, $(C_1—C_6)$-Alkyl, geradkettig oder verzweigt, Allyl, Methyallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1—C_{12})$-Alkyl, geradkettig oder verzweigt, Allyl, Methallyl, $(C_5—C_7)$-Cycloalkyl, $(C_5—C_7)$-Cycloalkyl-$(C_1—C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor,

R(4)' Wasserstoff,

m 1, 2,

n 0, 1, 2,

X ein $CH_2$-Gruppe,

R(5) eine der Gruppen

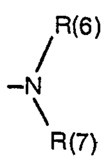

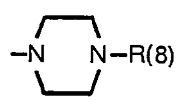

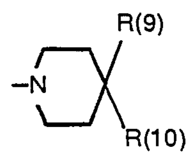

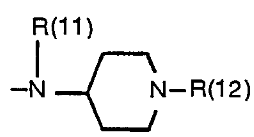

oder

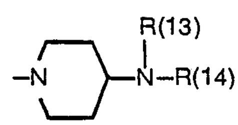

worin

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-$(C_1—C_4)$-alkyl, Benzhydryl oder Benzhydryl-$(C_1—C_4)$-alkyl, wobei die Phenylreste jeweils unsubstituiert oder durch einen, zwei oder drei Reste aus der Gruppe $(C_1—C_4)$-Alkyl, $(C_1—C_4)$-Alkoxy, $(C_1—C_2)$-Alkylendioxy, F, Cl, $CF_3$ oder Hydroxy substituiert sind, Pyridyl-$(C_1—C_4)$-alkyl,

R(8) Wasserstoff, $(C_1—C_6)$-Alkyl, geradkettig oder verzweigt, $(C_1—C_6)$-Alkanoyl, Phenyl, wobei der Phenylrest durch einen oder zwei Reste aus der Gruppe $(C_1—C_4)$-Alkyl, $(C_1—C_4)$-Alkoxy, $(C_1—C_2)$-Alkylendioxy, F, Cl, $CF_3$ oder Hydroxy substituiert sein kann, Phenyl-$(C_1—C_4)$-alkyl, Phenyl-$(C_3—C_5)$-alkenyl, Benzhydryl oder Benzhydryl-$(C_1—C_4)$-alkyl, Phenyl-$(C_1—C_4)$-alkanoyl oder Benzoyl, wobei die Phenylreste jeweils unsubstituiert oder durch einen, zwei oder drei Reste aus der Gruppe Methyl, Ethyl, Methoxy, Ethoxy, $(C_1—C_2)$-Alkylendioxy, F, Cl, $CF_3$ oder Hydroxy substituiert sind,

R(9) Phenyl, Phenyl-$(C_1—C_4)$-alkyl, wobei der Phenylrest jeweils unsubstituiert oder durch einen, zwei oder drei Reste aud der Gruppe $(C_1—C_4)$-Alkyl, $(C_1—C_4)$-Alkoxy, $(C_1—C_2)$-Alkylendioxy, F, Cl, $CF_3$ oder Hydroxy substituiert ist,

R(10) Wasserstoff, Hydroxy oder Methoxy

R(11), R(12), R(13) und R(14) gleich oder verschieden und Wasserstoff, $(C_1—C_8)$-Alkyl, $(C_1—C_6)$-Alkanoyl, Phenyl-$(C_1—C_4)$-alkyl, Benzhydryl oder Benzhydryl-$(C_1—C_4)$-alkyl, wobei die Phenylreste jeweils unsubstituiert oder durch einen, zwei oder drei Reste aus der Gruppe Methyl, Ethyl, Methoxy, Ethoxy, $(C_1—C_2)$-Alkylendioxy, F, Cl, $CF_3$ oder Hydroxy substituiert sind, Phenyl-$(C_1—C_4)$-alkanoyl oder Benzoyl, wobei die Phenylreste jeweils unsubstituiert oder durch einen, zwei oder drei Reste der Gruppe Methyl, Ethyl, Methoxy, Ethoxy, $(C_1—C_2)$-Alkylendioxy, F, Cl, $CF_3$ oder Hydroxy substituiert sind, bedeuten, sowie die Salze dieser Verbindungen der Formel I mit pharmazeutisch akzeptablen Säuren.

3. Verbindung nach einem der Ansprüche 1 und 2, dadurch gekennzeichnet, daß

R(1) Wasserstoff, Methyl, Methoxy, fluor oder Chlor,

R(1)' Wasserstoff, oder Methoxy

R(1)'' Wasserstoff

R(2) Wasserstoff, Methyl, Ethyl, Propyl, Isopropyl, Butyl, sec. Butyl, Isobutyl, Benzyl, Phenethyl,

R(3) Wasserstoff ($C_1$—$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl

R(4) Wasserstoff, Methoxy, Methyl,

R(4)' Wasserstoff

m 1, 2,

n 0, 1 oder 2,

X eine $CH_2$-Gruppe,

R(5) eine der folgenden Gruppen

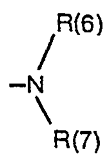
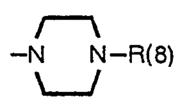
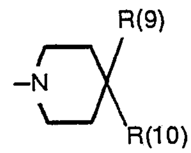

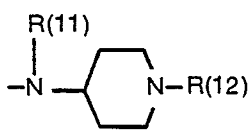
oder
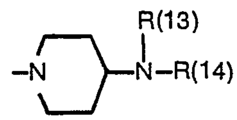

worin

R(6) Wasserstoff oder Methyl

R(7) Phenyl-($C_1$—$C_4$)-alkyl, Benzhydryl oder Benzhydryl-($C_1$—$C_4$)-alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist

R(8) ($C_1$—$C_6$)-Alkyl, geradkettig oder verzweigt, ($C_1$—$C_6$)-Alkanoyl, Phenyl, Phenyl-($C_1$—$C_4$)-alkyl, Benzhydryl oder Benzhydryl-($C_1$—$C_4$)-alkyl, Phenyl-($C_1$—$C_4$)-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist

R(10) Wasserstoff, Hydroxy oder Methoxy

R(11), R(12), R(13) und R(14) gleich oder verschieden sind und Wasserstoff, ($C_1$—$C_6$)-Alkyl, ($C_1$—$C_6$)-Alkanoyl, Phenyl-($C_1$—$C_4$)-alkyl, Benzhydryl oder Benzyhydryl-($C_1$—$C_4$)-alkyl, Phenyl-($C_1$—$C_4$)-alkanoyl oder Benzoyl, wobei die Phenylrest jeweils unsubstituiert oder durch einen, zwei oder drei Reste aus der Gruppe Methyl, Methoxy, Methylendioxy, Fluor, Chlor oder Hydroxy substituiert sind, bedeuten sowie die Salz dieser Verbindungen der Formel I mit pharmazeutisch akzeptablen Säuren.

4. Verfahren zur Herstellung einer Verbindung der Formel I, nach Anspruch 1, dadurch gekennzeichnet, daß man

a) eine Verbindung der Formel II,

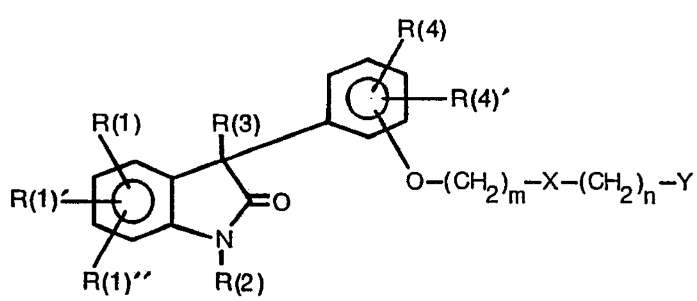
(II)

in welcher R(1), R(1)', R(1)'', R(2), R(3), R(4), R(4)', X, m und die gleiche Bedeutung wie in Formel I haben und in welcher Y ein Chlor-, Brom- oder Jodatom, ein Sulfonsäure-, ein Toluolsulfonyl- oder ein Trifluormethan- sulfonylrest ist, mit einer der Verbindungen der Formeln IIIa, IIIb, IIIc, IIIc oder IIIe

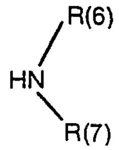 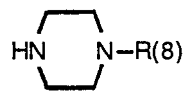 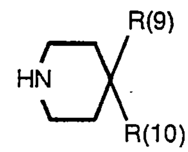

IIIa IIIb IIIc

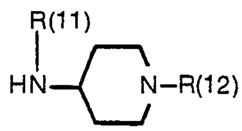 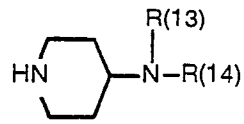

IIId IIIe

in welchen R(6), R(7), R(8), R(9), R(10), R(11), R(12), R(13) und R(14) die gleiche Bedeutung wie in Formel I haben, unter Bedingungen einer nucleophilen Substitution, umsetzt, oder daß man

b) eine Verbindung der Formel IV,

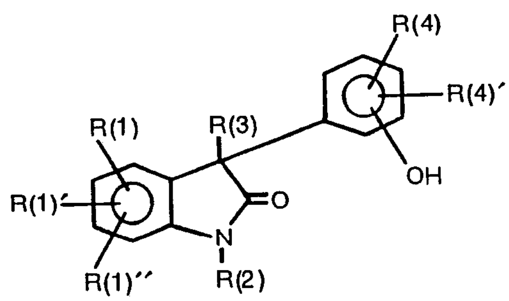

(IV)

in welcher R(1), R(1)', R(1)'', R(2), R(3), R(4) und R(4)' die gleiche Bedeutung wie in Formel I haben, mit einer Verbindung der Formel V,

$$Z—(CH_2)_m—X—(CH_2)_n—R(5) \quad V$$

in welcher Z gleich wie Y in Formel II definiert ist und in welcher R(5), X, m und n die gleiche Bedeutung wie in Formel I haben, umsetzt oder daß man

c) eine Verbindung der Formel VI

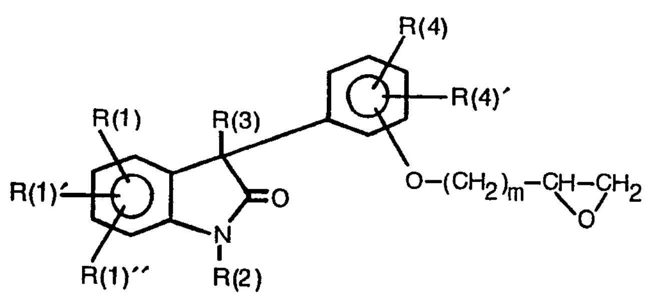

(VI)

in welcher R(1), R(1)', R(1)'', R(2), R(3), R(4), R(4)' und m die gleiche Bedeutung wie in Formel I haben, mit Aminen der Formel IIIa—IIIe umsetzt oder daß man

d) eine Verbindung der Formel VII

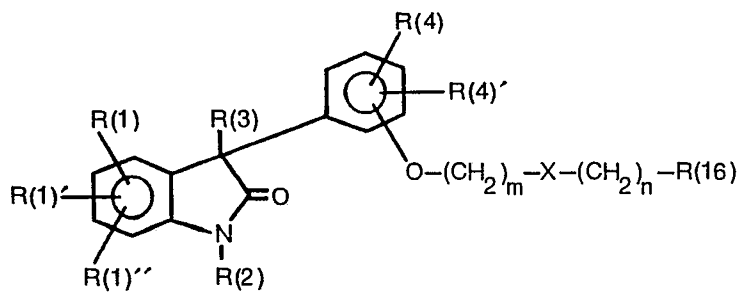

(VII)

in welcher R(1), R(1)', R(1)'', R(2), R(3), R(4), R(4)', X, m und n die gleiche Bedeutung wie in Formel I haben und R(16) eine der folgenden Gruppen

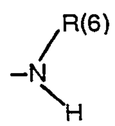

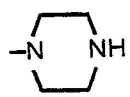 ,

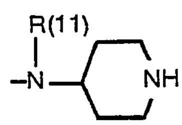 ,

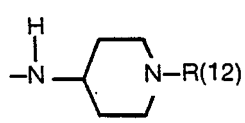 ,

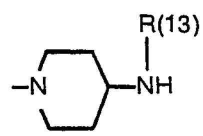

bedeuten, worin R(6), R(11), R(12) und R(13) die gleiche Bedeutung wie in Formel I haben, mit Alkylierungs- oder Acylierungsmitteln umsetzt.

5. Verbindung der allgemeinen Formel II

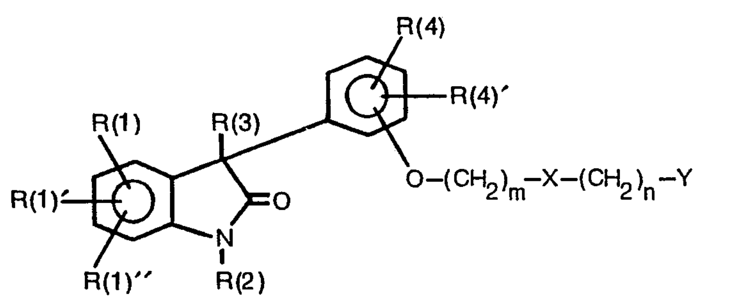

(II)

in welcher R(1), R(1)', R(1)'', R(2), R(3), R(4), R(4)', X, m und n die gleich Bedeutung wie in Formel I haben und in welcher Y ein Chlor-, Brom- oder Jodatom, ein Sulfonsäurerest, ein Toluolsulfonylrest oder ein Trifluormethansulfonylrest ist.

6. Verwendung einer Verbindung I nach Anspruch 1 zum Herstellen eines blutdrucksenkenden Mittels.

7. Verwendung einer Verbindung I nach Anspruch 1 als blutdrucksenkendes Mittel.

**Patentansprüche für den Vertragsstaat: AT**

1. Verfahren zur Herstellung einer Verbindung der Formel I,

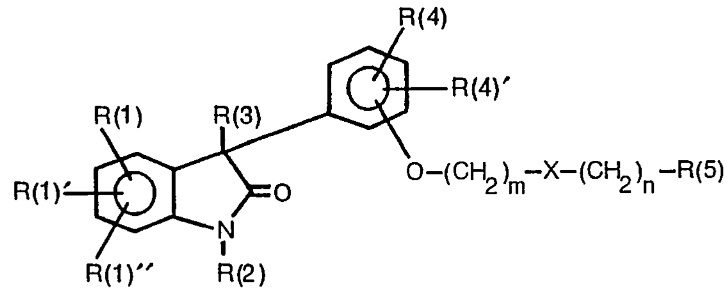

(I)

in welcher

R(1), R(1)' und R(1)'' gleich oder verschieden und voneinander unabhängig sind und Wasserstoff, ($C_1$—$C_4$)-Alkyl, ($C_1$—$C_3$)-Alkoxy, F, Cl, Br, $CF_3$, Nitro, Hydroxy, Acetamido oder Amino,

R(2) Wasserstoff, ($C_1$—$C_{10}$)-Alkyl, geradkettig oder verzweigt, ($C_3$—$C_{10}$)-Alkenyl, geradkettig oder verzweigt, Phenyl-($C_1$—$C_4$)-alkyl, wobei der Phenylring unsubstituiert oder durch einen, zwei oder drei Substituenten der Gruppe ($C_1$—$C_4$)-Alkyl, ($C_1$—$C_3$)-Alkoxy, F, Cl, $CF_3$, ($C_1$—$C_2$)-Alkylendioxy oder Nitro substituiert ist,

R(3) Wasserstoff, ($C_1$—$C_{15}$)-Alkyl, geradkettig oder verzweigt, ($C_3$—$C_{15}$)-Alkenyl, geradkettig oder verzweigt, ($C_4$—$C_8$)-Cycloalkyl, ($C_4$—$C_8$)-Cycloalkyl-($C_1$—$C_4$)alkyl, Phenyl oder Phenyl-($C_1$—$C_4$)-alkyl, wobei der Phenylrest unsubstituiert oder durch einen, zwei oder drei Substituenten der Gruppe ($C_1$—$C_4$)-Alkyl, ($C_1$—$C_3$)-Alkoxy, F, Cl, $CF_3$, ($C_1$—$C_2$)-Alkylendioxy oder Nitro substituiert ist

R(4) und R(4)' gleich oder verschieden und voneinander unabhängig Wasserstoff, ($C_1$—$C_4$)-Alkyl, ($C_1$—$C_3$)-Alkoxy, F, Cl, $CF_3$, Nitro, Hydroxy, Acetamido oder Amino

m 1, 2,

n 0, 1, 2,

X eine $CH_2$-Gruppe

R(5) eine der folgenden Gruppe

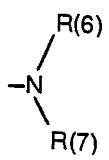

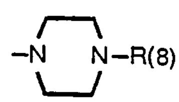

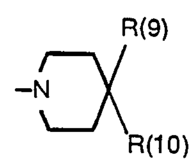

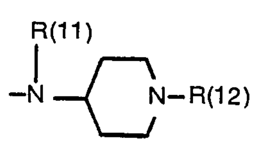

oder

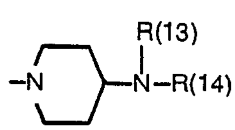

worin

R(6) und R(7) gleich oder verschieden voneinander unabhängig Wasserstoff, ($C_1$—$C_{10}$)-Alkyl, ($C_4$—$C_8$)-Cycloalkyl, ($C_4$—$C_8$)-Cycloalkyl-($C_1$—$C_4$)-alkyl, Pyridyl-($C_1$—$C_4$)-alkyl, Phenyl-($C_1$—$C_6$)-alkyl, Benzyhydryl oder Benzhydryl-($C_1$—$C_4$)-alkyl, wobei die Phenylrest jeweils unsubstituiert oder durch einen, zwei oder drei Reste aus der Gruppe ($C_1$—$C_4$)-Alkyl, ($C_1$—$C_4$)-Alkoxy, ($C_1$—$C_2$)-Alkylendioxy, F, Cl, Br, $CF_3$ oder Hydroxy substituiert sind,

R(8) Wasserstoff, ($C_1$—$C_{10}$)-Alkyl, geradkettig oder verzweigt, ($C_1$—$C_8$)-Alkanoyl, Pyridyl, Pyrimidinyl, Phenyl, Phenyl-($C_1$—$C_4$)-alkyl, Phenyl-($C_3$—$C_5$)-alkenyl, Benzhydryl oder Benzhydryl —($C_1$—$C_4$)-alkyl, Phenyl-($C_1$—$C_4$)-alkanoyl oder Benzoyl, wobei die Phenylreste jeweils unsubstituiert oder durch einen, zwei oder drei Reste aus der Gruppe ($C_1$—$C_4$)-Alkyl, ($C_1$—$C_4$)-Alkoxy, ($C_1$—$C_2$)-Alkylendioxy, F, Cl, Br, $CF_3$ oder Hydroxy substituiert sind,

R(9) Wasserstoff, $C_1$—$C_{10}$)-Alkyl, Phenyl, Phenyl-($C_1$—$C_4$)-alkyl, wobei der Phenylrest jeweils unsubstituiert oder durch einen, zwei oder drei Reste aus der Gruppe ($C_1$—$C_4$)-Alkyl, ($C_1$—$C_4$) Alkoxy, ($C_1$—$C_2$)-Alkylendioxy, F, Cl, Br, $CF_3$ oder Hydroxy substituiert ist,

R(10) Wasserstoff, Hydroxy, oder ($C_1$—$C_4$)-Alkoxy, und

R(11) und R(12) bzw, R(13) und R(14) gleich oder verschieden und voneinander unabhängig Wasserstoff, ($C_1$—$C_{10}$) alkyl, geradkettig oder verzweigt, ($C_1$—$C_6$)-Alkanoyl, Phenyl-($C_1$—$C_4$)-alkyl, Benzhydryl oder Benzhydryl-($C_1$—$C_4$)-alkyl, Phenyl-($C_1$—$C_4$)-alkanoyl, oder Benzoyl, wobei die Phenylreste jeweils unsubstituiert oder durch einen, zwei oder drei Rest aus der Gruppe ($C_1$—$C_4$)-Alkyl, ($C_1$—$C_4$)-Alkoxy, ($C_1$—$C_2$)-Alkylendioxy, F, Cl, Br, $CF_3$ oder Hydroxy substituiert sind, bedeuten, sowie die Salzen der Verbindungen der Formel I mit pharmazeutisch akzeptablen Säuren, dadurch gekennzeichnet, daß man

a) eine Verbindung der Formel II,

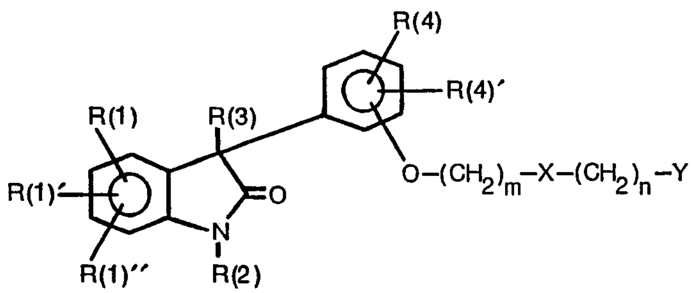

(II)

in welcher R(1), R(1)', R(1)'', R(2), R(3), R(4), R(4)', X, m und die gleiche Bedeutung wie in Formel I haben und in welcher Y ein Chlor-, Brom- oder Jodatom, ein Sulfonsäure-, ein Toluolsulfonyl- oder ein Trifluormethansulfonylrest ist, mit einer der Verbindungen der Formeln IIIa, IIIb, IIIc, IIIc oder IIIe

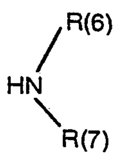

IIIa

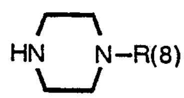

IIIb

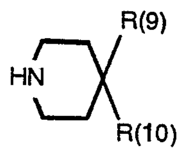

IIIc

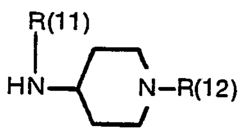

IIId

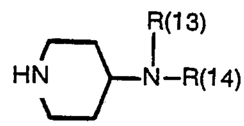

IIIe

in welchen R(6), R(7), R(8), R(9), R(10), R(11), R(12), R(13) und R(14) die gleiche Bedeutung wie in Formel I haben, unter Bedingungen einer nucleophilen Substitution, umsetzt, oder daß man

b) eine Verbindung der Formel IV,

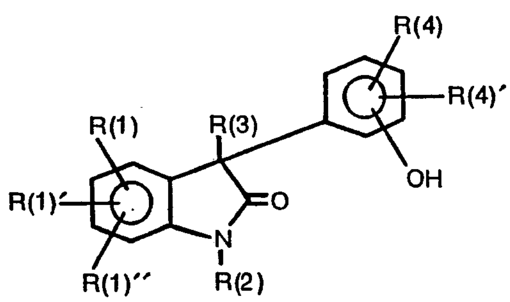

(IV)

in welcher R(1), R(1)', R(1)'', R(2), R(3), R(4) und R(4)' die gleiche Bedeutung wie in Formel I haben, mit einer Verbindung der Formel V,

$$Z—(CH_2)_m—X—(CH_2)_n—R(5) \qquad V$$

in welcher Z gleich wie Y in Formel II definiert ist und in welcher R(5), X, m und n die gleiche Bedeutung wie in Formel I haben, umsetzt oder daß man

c) eine Verbindung der Formel VI

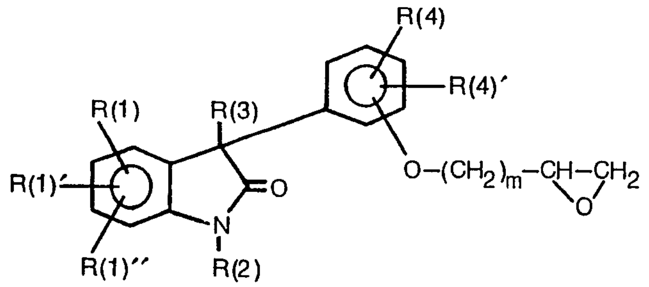

(VI)

in welcher R(1), R(1)', R(1)'', R(2), R(3), R(4), R(4)' und m die gleiche Bedeutung wie in Formel I haben, mit Aminen der Formel IIIa—IIIe umsetzt oder daß man

d) eine Verbindung der Formel VII

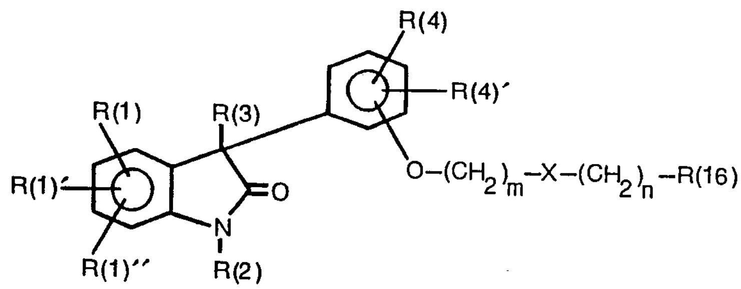

(VII)

in welcher R(1), R(1)', R(1)'', R(2), R(3), R(4), R(4)', X, m und n die gleiche Bedeutung wie in Formel I haben und R(16) eine der folgenden Gruppen

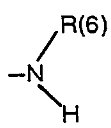

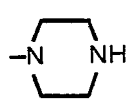
,

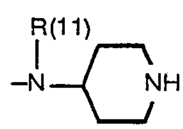
,

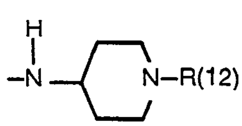
,

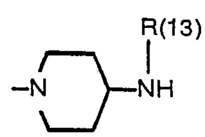

bedeuten, worin R(6), R(11), R(12) und R(13) die gleiche Bedeutung wie in Formel I haben, mit Alkylierungs- oder Acylierungsmitteln umsetzt.

2. Verfahren nach Anspruch 1, dadurch gekennzeichnet, daß

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)'' Wasserstoff,

R(2) Wasserstoff, ($C_1$—$C_6$)-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, ($C_1$—$C_{12}$)-Alkyl, geradkettig oder verzweigt, Allyl, Methallyl, ($C_5$—$C_7$)-Cycloalkyl, ($C_5$—$C_7$)-Cycloalkyl-($C_1$—$C_4$)-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

m 1, 2,

n 0, 1, 2,

X ein $CH_2$-Gruppe,

R(5) eine der Gruppen

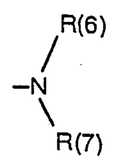
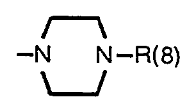
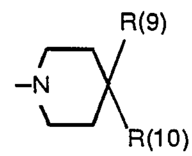

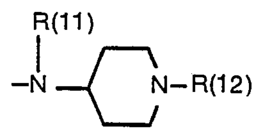
oder
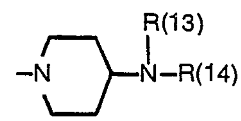

worin

R(6) Wasserstoff, Methyl, Ethyl, Propyl, Isopropyl,

R(7) Wasserstoff, Methyl, Ethyl, Propyl, Isopropyl, Cyclopentylethyl, Cyclohexylethyl, Phenyl-($C_1$—$C_4$)-alkyl, Benzhydryl oder Benzhydryl-($C_1$—$C_4$)-alkyl, wobei die Phenylreste jeweils unsubstituiert oder durch einen, zwei oder drei Reste aus der Gruppe ($C_1$—$C_4$)-Alkyl, ($C_1$—$C_4$)-Alkoxy, ($C_1$—$C_2$)-Alkylendioxy, F, Cl, $CF_3$ oder Hydroxy substituiert sind, Pyridyl-($C_1$—$C_4$)-alkyl,

R(8) Wasserstoff, ($C_1$—$C_6$)-Alkyl, geradkettig oder verzweigt, ($C_1$—$C_6$)-Alkanoyl, Phenyl, wobei der Phenylrest durch einen oder zwei Reste aus der Gruppe ($C_1$—$C_4$)-Alkyl, ($C_1$—$C_4$)-Alkoxy, ($C_1$—$C_2$)-Alkylendioxy, F, Cl, $CF_3$ oder Hydroxy substituiert sein kann, Phenyl-($C_1$—$C_4$)-alkyl, Phenyl-($C_3$—$C_5$)-alkenyl, Benzhydryl oder Benzylhydryl-($C_1$—$C_4$)-alkyl, Phenyl-($C_1$—$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, ($C_1$—$C_2$)-Alkylendioxy, F, Cl, $CF_3$ oder Hydroxy substituiert sind,

R(9) Phenyl, Phenyl-($C_1$—$C_4$)-alkyl, wobei der Phenylrest jeweils unsubstituiert oder durch einen, zwei oder drei Reste aus der Gruppe ($C_1$—$C_4$)-Alkyl, ($C_1$—$C_4$)-Alkoxy, ($C_1$—$C_2$)-Alkylendioxy, F, Cl, $CF_3$ oder Hydroxy substituiert ist,

R(10) Wasserstoff, Hydroxy oder Methoxy

R(11), R(12), R(13) und R(14) gleich oder verschieden und Wasserstoff, ($C_1$—$C_8$)-Alkyl, ($C_1$—$C_6$)-Alkanoyl, Phenyl-($C_1$—$C_4$)-alkyl, Benzhydryl oder Benzhydryl-($C_1$—$C_4$)-alkyl, wobei die Phenylreste jeweils unsubstituiert oder durch einen, zwei oder drei Reste aus der Gruppe Methyl, Ethyl, Methoxy, Ethoxy, ($C_1$—$C_2$)-Alkylendioxy, F, Cl, $CF_3$ oder Hydroxy substituierts ein können, Phenyl-($C_1$—$C_4$)-alkanoyl oder Benzoyl, wobei die Phenylreste jeweils unsubstituiert oder durch einen, zwei oder drei Reste der Gruppe Methyl, Ethyl, Methoxy, Ethoxy, ($C_1$—$C_2$)-Alkylendioxy, F, Cl, $CF_3$ oder Hydroxy substituiert sind, bedeuten.

3. Verfahren nach einem der Ansprüche 1 und 2, dadurch gekennzeichnet, daß

R(1) Wasserstoff, Methyl, Methoxy, Fluor oder Chlor,

R(1)' Wasserstoff, oder Methoxy

R(1)'' Wasserstoff

R(2) Wasserstoff, Methyl, Ethyl, Propyl, Isopropyl, Butyl, sec. Butyl, Isobutyl, Benzyl, Phenethyl,

R(3) Wasserstoff ($C_1$—$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl

R(4) Wasserstoff, Methoxy, Methyl, Chlor, Nitro oder Hydroxy

R(4)' Wasserstoff

m 1, 2,

n 0, 1 oder 2,

X eine $CH_2$-Gruppe,

R(5) eine der folgenden Gruppen

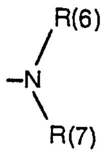

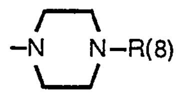

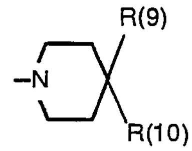

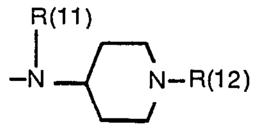

oder

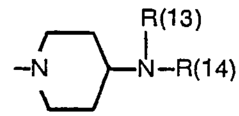

worin

R(6) Wasserstoff oder Methyl

R(7) Phenyl-($C_1$—$C_4$)-alkyl, Benzhydryl oder Benzhydryl-($C_1$—$C_4$)-alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist

R(8) ($C_1$—$C_6$)-Alkyl, geradkettig oder verzweigt, ($C_1$—$C_6$)-Alkanoyl, Phenyl, Phenyl-($C_1$—$C_4$)-alkyl, Benzhydryl oder Benzhydryl-($C_1$—$C_4$)-alkyl, Phenyl-($C_1$—$C_4$)-alkanoyl oder Benzoyl, wobei die Phenylrest jeweils unsubstituiert oder durch einen, zwei oder drei Reste aus der Gruppe Methyl, Methoxy, Ethoxy, Methylendioxy, Fluor, Chlor oder Hydroxy substituiert sind

R(9) Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist

R(10) Wasserstoff, Hydroxy oder Methoxy

R(11), R(12), R(13) und R(14) gleich oder verschieden sind und Wasserstoff, ($C_1$—$C_6$)-Alkyl, ($C_1$—$C_6$)-Alkanoyl, Phenyl-($C_1$—$C_4$)-alkyl, Benzhydryl oder Benzyhydryl-($C_1$—$C_4$)-alkyl, Phenyl-($C_1$—$C_4$)-alkanoyl oder Benzoyl, wobei die Phenylrest jeweils unsubstituiert oder durch einen, zwei oder drei Reste aus der Gruppe Methyl, Methoxy, Methylendioxy, Fluor, Chlor oder Hydroxy substituiert sind, bedeuten.

4. Verfahren zur Herstellung einer Verbindung der allgemeinen Formel II

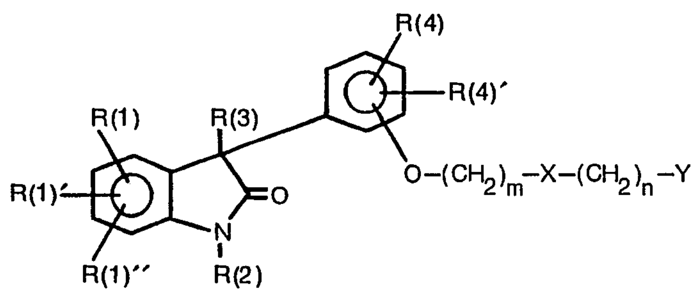

(II)

in welcher R(1), R(1)', R(1)'', R(2), R(3), R(4), R(4)', X, m und n die gleich Bedeutung wie in Formel I haben und in welcher Y wie in Anspruch 1 definiert ist, dadurch gekennzeichnet, daß man substituierte Aniline der Formel VIII,

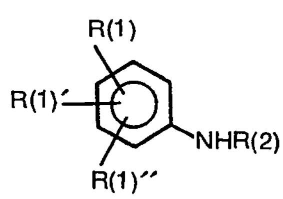

(VIII)

in welcher R(1), R(1)', R(1)'' und R(2) die gleiche Bedeutung wie in Formel I haben, in welcher Formel VIII mindestend eine Orthostellung zur Aminogruppe frei sein muß, umsetzt mit einer Verbindung der Formel IX

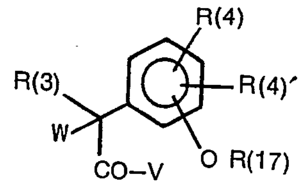

(IX)

in welcher R(3), R(4) und R(4)' die gleiche Bedeutung wie in Formel I haben, R(17) eine unter milden Bedingungen abspaltbare Schutzgruppe darstellt, V OH, Cl oder O($C_1$—$C_4$)-Alkyl und W OH, OAc, Chlor oder Brom bedeuten, nach den allgemein bekannten Methoden für die Synthese von Amiden aus Aminen und Carbonsäurederivaten, wobei Verbindungen der Formel X

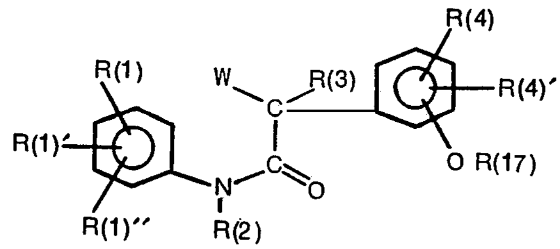

(X)

gebildet werden, in welchen R(1), R(1)', R(1)'', R(2), R(3), R(4) und R(4)' die gleich Bedeutung wie in Formel I, R(17) und W die gleiche Bedeutung wie in Formel IX besitzen, daß man sofern W in Formel X eine O Acetylgruppe bedeutet, diese vor der folgenden Cylisierungsreaktion in eine freie Hydroxylgruppe verwandelt, daß man dann die Verbindungen der Formel X mit wasserabspaltenden Mitteln zu Verbindungen der formel XI

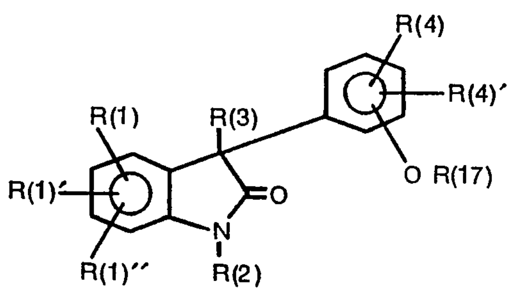

(XI)

worin R(1), R(1)', R(1)'', R(2), R(3), R(4) und R(4)' dieselbe Bedeutung haben wie in Formel I und R(17) dieselbe Bedeutung hat wie in Formel IX umsetzt, daß man sodann durch Abspaltung der Schutzgruppe R(17) unter geeigneten Bedingungen, die Verbindungen der Formel IV

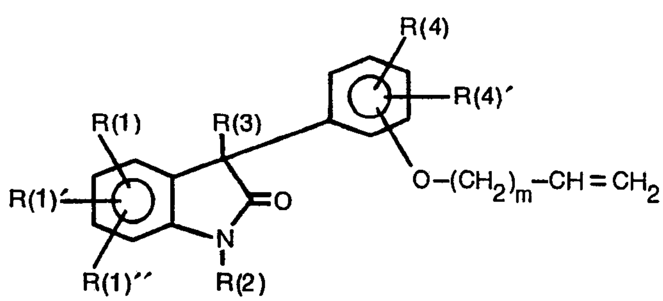

(XV)

herstellt, und daß man aus diesen durch Umsetzung mit Verbindungen der Formel XIII

$$Q—(CH_2)_m—X—(CH_2)_n—Y \qquad XIII$$

in welcher x, m und n die gleiche Bedeutung wie in Formel I, Y die gleiche Bedeutung wie in Formel II Q gleich definiert ist wie Y in Formel II in Gegenwart von Basen Verbindungen der Formel II herstellt.

5. Verfahren zum Herstellen eines Medikaments zur Behandlung des Bluthochdrucks, dadurch gekennzeichnet, daß man eine Verbindung I nach Anspruch 1 mit pharmazeutisch üblichen Zusatzstoffen vermischt.

**Revendications pour les Etats contractants: BE CH DE FR GB IT LI LU NL SE**

1. Composé de formule I

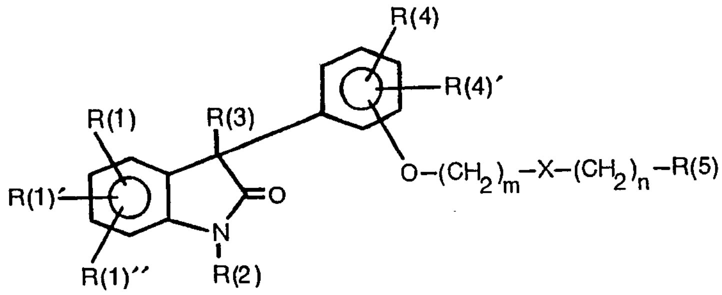

(I)

dans laquelle

R(1), R(1)' et R(1)'' sont identiques ou différents et indépendants les uns des autres, et représentent un atome d'hydrogène ou de F, Cl, Br, ou un groupe alkyle en $C_1$—$C_4$, alcoxy en $C_1$-$C_3$, $CF_3$, nitro, hydroxy, acétamido ou amino;

R(2) représente un atome d'hydrogène ou un radical alkyle en $C_1$—$C_{10}$ à chaîne droite ou ramifiée, alcényle en $C_3$—$C_{10}$ à chaîne droite ou ramifiée, phényl-alkyle($C_1$—$C_4$), le noyau phényle n'étant pas substitué ou étant substitué par un, deux ou trois substituants choisis parmi F, Cl, $CF_3$ et des groupes alkyle en $C_1$—$C_4$, alcoxy en $C_1$—$C_3$, alkylène($C_1$—$C_2$)-dioxy ou nitro;

R(3) représente un atome d'hydrogène ou un radical alkyle en $C_1$—$C_{15}$ à chaîne droite ou ramifiée, alcényle en $C_3$—$C_{15}$ à chaîne droite ou ramifiée, cycloalkyle en $C_4$—$C_8$, cycloalkyl($C_4$—$C_8$)-alkyle($C_1$—$C_4$), phényle ou phénylalkyle($C_1$—$C_4$), le fragment phényle n'étant pas substitué ou étant substitué par un, deux ou trois substituants choisis parmi F, Cl, $CF_3$ et des groupes alkyles en $C_1$—$C_4$, alcoxy en $C_1$—$C_3$, alkylène($C_1$—$C_2$)-dioxy ou nitro;

R(4) et R(4)' sont identiques ou différents, et représentent, indépendamment l'un de l'autre, un atome d'hydrogène ou de F, Cl, ou un groupe alkyle en $C_1$—$C_4$, alcoxy en $C_1$—$C_3$, $CF_3$;

m est 1, 2;

n est 0, 1, 2;

X représente le groupe $CH_2$;

R(5) représente l'un des groupes suivants

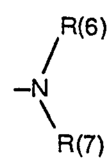

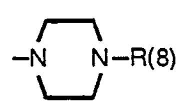

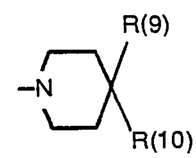

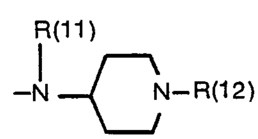

ou

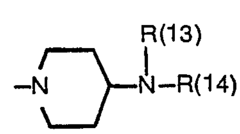

dans lesquels

R(6) et R(7) sont identiques ou différents, et représentent indépendamment l'un de l'autre un atome d'hydrogène ou un radical alkyle en $C_1$—$C_{10}$, cycloalkyle en $C_4$—$C_8$, cycloalkyl($C_4$—$C_8$)-alkyle($C_1$—$C_4$), pyridyl-alkyle($C_1$—$C_4$), phényl-alkyle($C_1$—$C_6$), benzhydryle ou benzhydrylalkyle($C_1$—$C_4$), les fragments phényle n'étant pas substitués ou étant substitués par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)dioxy ou hydroxy;

R(8) représente un atome d'hydrogène ou un radical alkyle en $C_1$—$C_{10}$ à chaîne droite ou ramifiée, alcanoyle en $C_1$—$C_8$, pyridyle, pyrimidinyle, phényle, phényl-alkyle($C_1$—$C_4$), phényl-alcényle($C_3$—$C_5$), benzhydryle ou benzhydrylalkyle($C_1$—$C_4$), phényl-alcanoyle($C_1$—$C_4$) ou benzoyle, les fragments phényle n'étant pas substitués ou étant substitués par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)-dioxy ou hydroxy;

R(9) représente un atome d'hydrogène ou un radical alkyle en $C_1$—$C_{10}$, phényle, phényl-alkyle($C_1$—$C_4$), le fragment phényle n'étant pas substitué ou étant substitué par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)-dioxy ou hydroxy;

R(10) représente un atome d'hydrogène ou un groupe hydroxy ou alcoxy en $C_1$—$C_4$; et

R(11) et R(12) ou respectivement R(13) et R(14) sont identiques ou différents et représentent, indépendamment l'un de l'autre, un atome d'hydrogène ou un groupe alkyle en $C_1$—$C_{10}$ à chaîne droite ou ramifiée, alcanoyle en $C_1$—$C_6$, phényl-alkyle($C_1$—$C_4$), benzhydryle ou benzhydryl-alkyle($C_1$—$C_4$), phényl-alcanoyle($C_1$—$C_4$), ou benzoyle, les fragments phényle n'étant pas substitués ou étant substitués par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)-dioxy ou hydroxy;

ainsi que les sels des composés de formule I avec des acides pharmaceutiquement acceptables.

2. Composé selon la revendication 1, caractérisé en ce que

R(1) et R(1)' sont identiques ou différents, et représentent, indépendamment l'une de l'autre, un atome d'hydrogène ou de fluor ou de chlore, ou un groupe méthyle, éthyle, méthoxy, éthoxy, $CF_3$, nitro ou acétamido;

R(1)'' représente un atome d'hydrogène;

R(2) représente un atome d'hydrogène ou un radical alkyle en $C_1$—$C_6$ à chaîne droite ou ramifiée, allyle, méthallyle, benzyle, phénéthyle, 4-méthoxybenzyle, 3,4-diméthoxybenzyle, 3,4,5-triméthoxybenzyle, 3,4-méthylènedioxybenzyle;

R(3) représente un atome d'hydrogène ou un radical alkyle en $C_1$—$C_{12}$ à chaîne droite ou ramifiée, allyle, méthallyle, cycloalkyle en $C_5$—$C_7$, cycloalkyl($C_5$—$C_7$)-alkyle($C_1$—$C_4$), benzyle, méthylbenzyle, fluorobenzyle, méthoxybenzyle, diméthoxybenzyle, phényléthyle;

R(4) représente un atome d'hydrogène ou de chlore, ou un groupe méthyle, méthoxy, éthoxy;

R(4)' représente un atome d'hydrogène;

m est 1, 2;

n, est 0, 1, 2;

X représente le groupe $CH_2$;

R(5) représente l'un des groupes

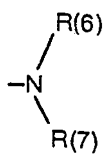

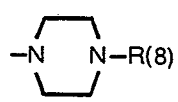

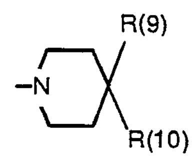

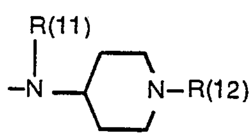

ou

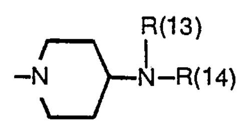

dans lesquels

R(6) représente un atome d'hydrogène ou le radical méthyle, éthyle, propyle, isopropyle;

R(7) représente un atome d'hydrogène ou le radical méthyle, éthyle, propyle, isopropyle, cyclopentyléthyle, cyclohexyléthyle, phényl-alkyle($C_1$—$C_4$), benzhydryle ou benzhydryl-alkyle($C_1$—$C_4$), les fragments phényle n'étant pas substitués ou étant substitués par un, deux ou trois substituants choisis parmi F, Cl, $CF_3$ ou des groupes alkyles en $C_1$—$C_4$, alcoxy en $C_1$—$C_4$, alkylène($C_1$—$C_2$)-dioxy ou hydroxy, ou un radical pyridyl-alkyle($C_1$—$C_4$);

R(8) représente un atome d'hydrogène ou un radical alkyle en $C_1$—$C_6$ à chaîne droite ou ramifiée, alcanoyle en $C_1$—$C_6$, phényle, le fragment phényle pouvant être substitué par un ou deux substituants choisis parmi F, Cl, $CF_3$ ou des radicaux alkyle en $C_1$—$C_4$, alcoxy en $C_1$—$C_4$, alkylène($C_1$—$C_2$)-dioxy ou hydroxy, un radical phényl-alkyle($C_1$—$C_4$), phényl-alcényle($C_3$—$C_5$), benzhydryle ou benzhydryl-alkyle($C_1$—$C_4$), phényl-alcanoyle($C_1$—$C_4$) ou benzoyle, les fragments phényle n'étant pas substitués ou étant substitués par un, deux ou trois substituants choisis parmi F, Cl, $CF_3$ ou des groupes méthyle, éthyle, méthoxy, éthoxy, alkylène($C_1$—$C_2$)-dioxy ou hydroxy;

R(9) représente un radical phényle, phényl-alkyle($C_1$—$C_4$), le fragment phényle n'étant pas substitué ou étant substitué par un, deux ou trois substituants choisis parmi F, Cl, $CF_3$ ou des groupes alkyle en $C_1$—$C_4$, alcoxy en $C_1$—$C_4$, alkylène($C_1$—$C_2$)-dioxy ou hydroxy;

R(10) représente un atome d'hydrogène ou le groupe hydroxy ou méthoxy;

R(11), R(12), R(13) et R(14) sont identiques ou différents et représentent un atome d'hydrogène ou un radical alkyle en $C_1$—$C_8$, alcanoyle en $C_1$—$C_6$, phényl-alkyle($C_1$—$C_4$), benzhydryle ou benzhydryl-alkyle($C_1$—$C_4$), les fragments phényle n'étant pas substitués ou pouvant être substitués par un, deux ou trois substituants choisis parmi F, Cl, $CF_3$ ou des groupes méthyle, éthyle, méthoxy, éthoxy, alkylène($C_1$—$C_2$)-dioxy ou hydroxy, un radical phényl-alcanoyle($C_1$—$C_4$) ou benzoyle, les fragments phényle n'étant pas substitués ou étant substitués par un, deux ou trois substituants choisis parmi F, Cl, $CF_3$ ou des groupes méthyle, éthyle, méthoxy, éthoxy, alkylène($C_1$—$C_2$)-dioxy ou hydroxy;

ainsi que les sels de ces composés de formule I avec des acides pharmaceutiquement acceptables.

3. Composé selon l'une des revendications 1 et 2, caractérisé en ce que

R(1) représente un atome d'hydrogène, de fluor ou de chlore, ou le groupe méthyle ou méthoxy;

R(1)' représente un atome d'hydrogène ou le groupe méthoxy;

R(1)'' représente un atome d'hydrogène;

R(2) représente un atome d'hydrogène ou le groupe méthyle, éthyle, propyle, isopropyle, butyle, sec-butyle, isobutyle, benzyle, phénéthyle;

R(3) représente un atome d'hydrogène ou un radical alkyle en $C_1$—$C_{12}$ à chaîne droite ou ramifiée, cyclopentyle, cyclohexyle, cyclopentylméthyle, cyclohexylméthyle, allyle, méthallyle, benzyle, méthylbenzyle, fluorobenzyle, méthoxybenzyle, diméthoxybenzyle, phényléthyle;

R(4) représente un atome d'hydrogène ou le groupe méthoxy ou méthyle;

R(4)' représente un atome d'hydrogène;

m est 1, 2;

n est 0, 1 ou 2;

X représente le groupe $CH_2$;

R(5) représente l'un des groupes suivants

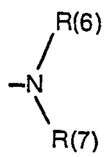
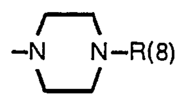
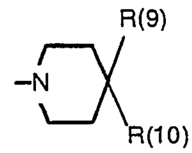

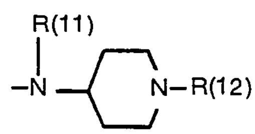
ou
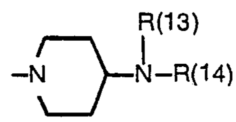

dans lesquels

R(6) représente un atome d'hydrogène ou le groupe méthyle;

R(7) représente un radical phényl-alkyle($C_1$—$C_4$), benzhydryle ou benzhydryl-alkyle($C_1$—$C_4$), le fragment phényle n'étant pas substitué ou étant substitué par un, deux ou trois substituants choisis parmi des atomes de fluor et de chlore ou les groupes méthyle, méthoxy, méthylènedioxy ou hydroxy;

R(8) représente un radical alkyle en $C_1$—$C_6$ à chaîne droite ou ramifiée, alcanoyle en $C_1$—$C_6$, phényle, phénylalkyle($C_1$—$C_4$), benzhydryle ou benzhydryl-alkyle($C_1$—$C_4$), phényl-alcanoyle($C_1$—$C_4$) ou benzoyle, les radicaux phényle n'étant pas substitués ou étant substitués par un, deux ou trois substituants choisis parmi des atomes de fluor ou de chlore ou les groupes méthyle, méthoxy, éthoxy, méthylènedioxy ou hydroxy;

R(9) représente un radical phényle, le radical phényle n'étant pas substitué ou étant substitué par un, deux ou trois substituants choisis parmi des atomes de fluor ou de chlore ou les groupes méthyle, méthoxy, méthylènedioxy ou hydroxy;

R(10) représente un atome d'hydrogène ou le groupe hydroxy ou méthoxy;

R(11), R(12), R(13) et R(14) sont identiques ou différents et représentent un atome d'hydrogène ou un radical alkyle en $C_1$—$C_6$, alcanoyle en $C_1$—$C_6$, phényl-alkyle($C_1$—$C_4$), benzhydryle ou benzhydryl-alkyle($C_1$—$C_4$), phénylalcanoyle($C_1$—$C_4$) ou benzoyle, les fragments phényle n'étant pas substitués ou étant substitués par un, deux ou trois substituants choisis parmi des atomes de fluor ou de chlore ou les groupes méthyle, méthoxy, méthylènedioxy ou hydroxy;

ainsi que les sels de ces composés de formule I avec des acides pharmaceutiquement acceptables.

4. Procédé pour la préparation d'un composé de formule I selon la revendication 1, caractérisé en ce que

a) on fait réagir un composé de formule II

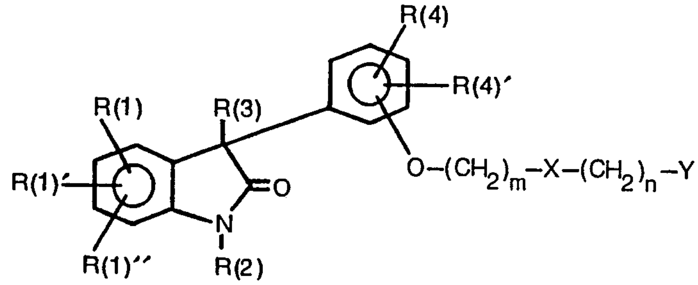
(II)

dans laquelle R(1), R(1)', R(1)'', R(2), R(3), R(4), R(4)', X, m et n ont les mêmes significations que dans la formule I, et dans laquelle Y est un atome de chlore, brome ou iode ou un groupe sulfo, toluènesulfonyle ou trifluorométhanesulfonyle, avec l'un des composés de formules IIIa, IIIb, IIIc, IIId ou IIIe

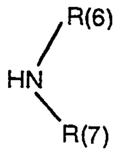

IIIa

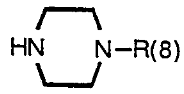

IIIb

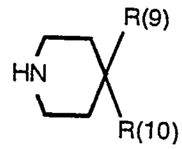

IIIc

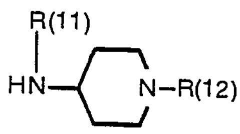

IIId

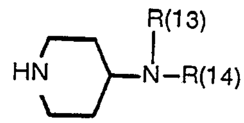

IIIe

dans lesquelles R(6), R(7), R(8), R(9), R(10), R(11), R(12), R(13) et R(14) ont les mêmes significations que dans la formule I, dans des conditions d'une substitution nucléophile, ou en ce que

b) on fait réagir un composé de formule IV

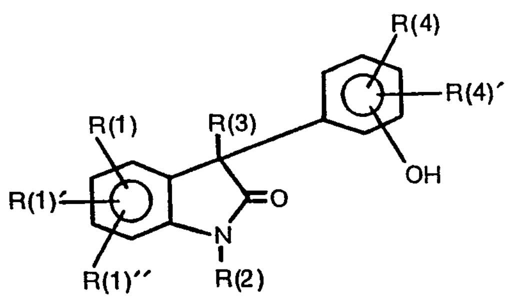

(IV)

dans laquelle R(1), R(1)', R(1)'', R(2), R(3), R(4) et R(4)' ont les mêmes significations que dans la formule I, avec un composé de formule V,

$$Z—(CH_2)_m—X—(CH_2)_n—R(5) \qquad (V)$$

dans laquelle Z a la même définition que dans la formule II et dans laquelle R(5), X, m et n ont les mêmes significations que dans la formule I, ou en ce que

c) on fait réagir un composé de formule VI

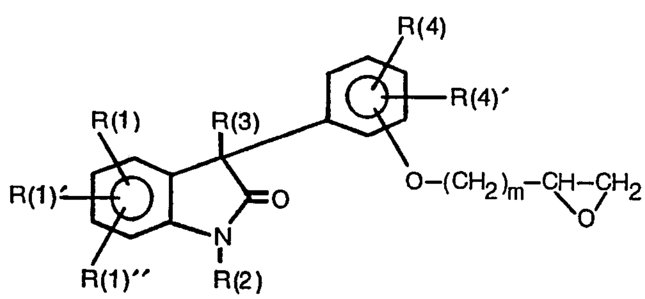

(VI)

dans laquelle R(1), R(1)', R(1)'', R(2), R(3), R(4), R(4)' et m ont les mêmes significations que dans la formule I, avec des amines de formules IIIa-IIIe, ou en ce que

d) on fait réagir un composé de formule VII

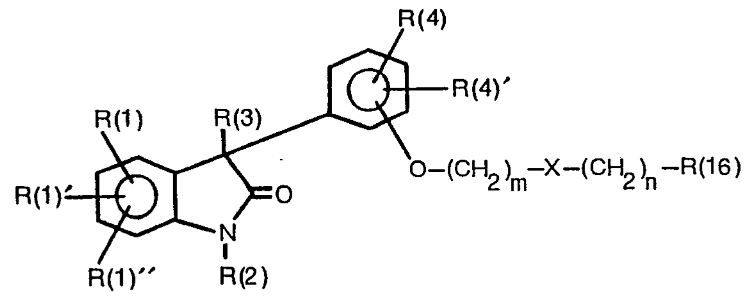

(VII)

dans laquelle R(1), R(1)', R(1)'', R(2), R(3), R(4), R(4)', X, m et n ont les mêmes significations que dans la formule I, et R(16) est l'un des groupes suivants

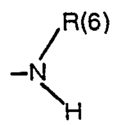

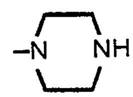 ,

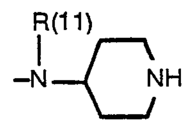 ,

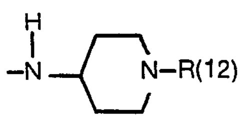 ,

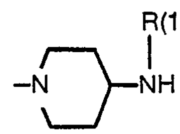

dans lesquels R(6), R(11), R(12) et R(13) ont les mêmes significations que dans la formule I, avec des agents d'alkylation ou d'alcylation.

5. Composé de formule générale II

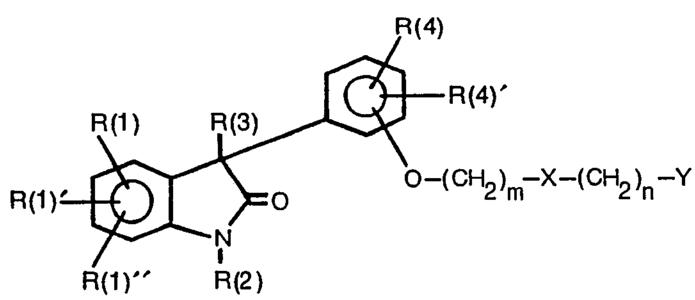

(II)

dans laquelle R(1), R(1)', R(1)'', R(2), R(3), R(4), R(4)', X, m et n ont les mêmes significations que dans la formule I et dans laquelle Y est un atome de chlore, brome ou iode ou un groupe sulfonyle, toluènesulfonyle ou trifluorométhanesulfonyle.

6. Utilisation d'un composé I selon la revendication 1, pour la fabrication d'un agent hypotenseur.

7. Utilisation d'un composé I selon la revendication 1 en tant qu'agent hypotenseur.

**Revendications pour l'Etat contractant: AT**

1. Procédé pour la préparation d'un composé de formule I

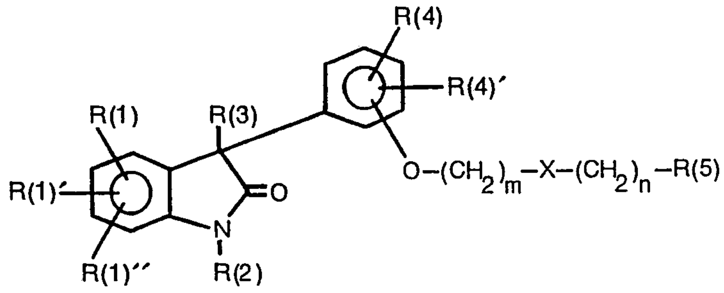

(I)

dans laquelle

R(1), R(1)' et R(1)'' sont identiques ou différents et indépendants les uns des autres, et représentent un atome d'hydrogène ou de F, Cl, Br, ou un groupe alkyle en $C_1$—$C_4$, alcoxy en $C_1$–$C_3$, $CF_3$, nitro, hydroxy, acétamido ou amino;

R(2) représente un atome d'hydrogène ou un radical alkyle en $C_1$—$C_{10}$ à chaîne droite ou ramifiée, alcényle en $C_3$—$C_{10}$ à chaîne droite ou ramifiée, phényl-alkyle($C_1$—$C_4$), le noyau phényle n'étant pas substitué ou étant substitué par un, deux ou trois substituants choisis parmi F, Cl, $CF_3$ et des groupes alkyle en $C_1$—$C_4$, alcoxy en $C_1$—$C_3$, alkylène($C_1$—$C_2$)-dioxy ou nitro;

R(3) représente un atome d'hydrogène ou un radical alkyle en $C_1$—$C_{15}$ à chaîne droite ou ramifiée, alcényle en $C_3$—$C_{15}$ à chaîne droite ou ramifiée, cycloalkyle en $C_4$—$C_8$, cycloalkyl($C_4$—$C_8$)-alkyle($C_1$—$C_4$), phényle ou phénylalkyle($C_1$—$C_4$), le fragment phényle n'étant pas substitué ou étant substitué par un, deux ou trois substituants choisis parmi F, Cl, $CF_3$ et des groupes alkyles en $C_1$—$C_4$, alcoxy en $C_1$—$C_3$, alkylène($C_1$—$C_2$)-dioxy ou nitro;

R(4) et R(4)' sont identiques ou différents, et représentent, indépendamment l'un de l'autre, un atome d'hydrogène ou de F, Cl, ou un groupe alkyle en $C_1$—$C_4$, alcoxy en $C_1$—$C_3$, $CF_3$; nitro, hydroxy, acétamido ou amino:

m est 1, 2;

n est 0, 1, 2;

X représente le groupe $CH_2$;

R(5) représente l'un des groupes suivants

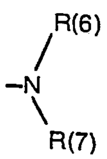

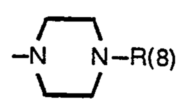

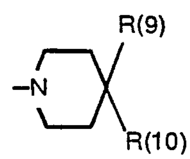

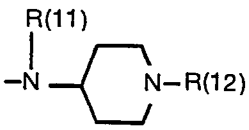

ou

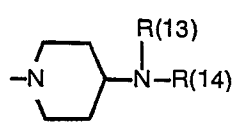

dans lesquels

R(6) et R(7) sont identiques ou différents, et représentent indépendamment l'un de l'autre un atome d'hydrogène ou un radical alkyle en $C_1$—$C_{10}$, cycloalkyle en $C_4$—$C_8$, cycloalkyl($C_4$—$C_8$)-alkyle($C_1$—$C_4$), pyridyl-alkyle($C_1$—$C_4$), phényl-alkyle($C_1$—$C_6$), benzhydryle ou benzhydrylalkyle($C_1$—$C_4$), les fragments phényle n'étant pas substitués ou étant substitués par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)dioxy ou hydroxy;

R(8) représente un atome d'hydrogène ou un radical alkyle en $C_1$—$C_{10}$ à chaîne droite ou ramifiée, alcanoyle en $C_1$—$C_8$, pyridyle, pyrimidinyle, phényle, phényl-alkyle($C_1$—$C_4$), phényl-alcényle($C_3$—$C_5$), benzhydryle ou benzhydrylalkyle($C_1$—$C_4$), phényl-alcanoyle($C_1$—$C_4$) ou benzoyle, les fragments phényle n'étant pas substitués ou étant substitués par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)-dioxy ou hydroxy;

R(9) représente un atome d'hydrogène ou un radical alkyle en $C_1$—$C_{10}$, phényle, phényl-alkyle($C_1$—$C_4$), le fragment phényle n'étant pas substitué ou étant substitué par un, deux ou trois substituants choisis parmi F, Cl, Br, $CF_3$ ou des groupes alkyle en $C_1$—$C_4$, alcoxy en $C_1$—$C_4$, alkylène($C_1$—$C_2$)-dioxy ou hydroxy;

R(10) représente un atome d'hydrogène ou un groupe hydroxy ou alcoxy en $C_1$—$C_4$; et

R(11) et R(12) ou respectivement R(13) et R(14) sont identiques ou différents et représentent, indépendamment l'un de l'autre, un atome d'hydrogène ou un groupe alkyle en $C_1$—$C_{10}$ à chaîne droite ou ramifiée, alcanoyle en $C_1$—$C_6$, phényl-alkyle($C_1$—$C_4$), benzhydryle ou benzhydryl-alkyle($C_1$—$C_4$), phényl-alcanoyle($C_1$—$C_4$), ou benzoyle, les fragments phényle n'étant pas substitués ou étant substitués par un, deux ou trois substituants choisis parmi F, CL, Br, $CF_3$ ou des groupes alkyle en $C_1$—$C_4$, alcoxy en $C_1$—$C_4$, alkylène($C_1$—$C_2$)-dioxy ou hydroxy;

ainsi que des sels des composés de formule I avec des acides pharmaceutiquement acceptables, caractérisé en ce que

a) on fait réagir un composé de formule II

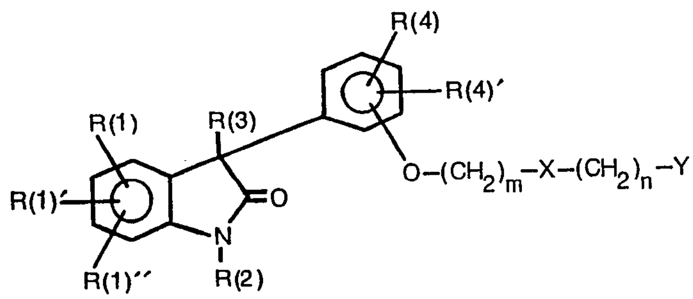

(II)

dans laquelle R(1), R(1)', R(1)'', R(2), R(3), R(4), R(4)', X, m et n ont les mêmes significations que dans la formule I, et dans laquelle Y est un atome de chlore, brome ou iode ou un groupe sulfo, toluènesulfonyle ou trifluorométhanesulfonyle, avec l'un des composés de formules IIIa, IIIb, IIIc, IIId ou IIIe

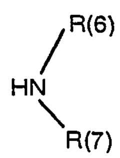

IIIa

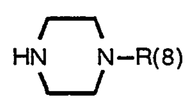

IIIb

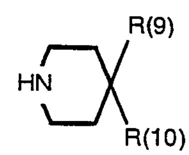

IIIc

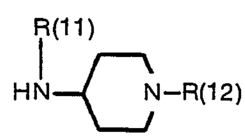

IIId

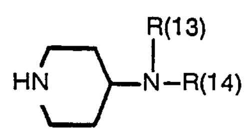

IIIe

dans lesquelles R(6), R(7), R(8), R(9), R(10), R(11), R(12), R(13) et R(14) ont les mêmes significations que dans la formule I, dans des conditions d'une substitution nucléophile, ou en ce que

b) on fait réagir un composé de formule IV

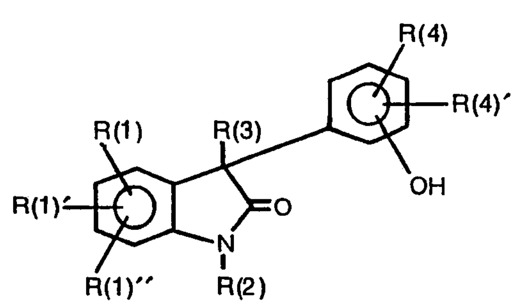

(IV)

dans laquelle R(1), R(1)', R(1)'', R(2), R(3), R(4) et R(4)' ont les mêmes significations que dans la formule I, avec un composé de formule V,

$$Z—(CH_2)_m—X—(CH_2)_n—R(5) \quad (V)$$

dans laquelle Z a la même définition que dans la formule II et dans laquelle R(5), X, m et n ont les mêmes significations que dans la formule I, ou en ce que

c) on fait réagir un composé de formule VI

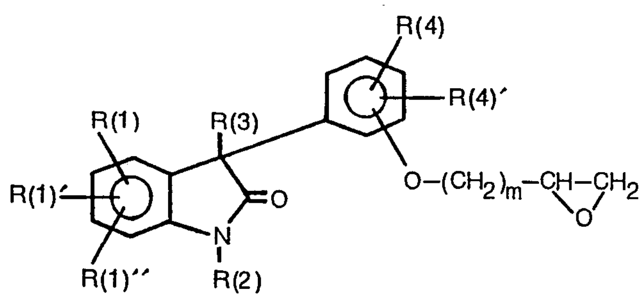

(VI)

dans laquelle R(1), R(1)', R(1)'', R(2), R(3), R(4), R(4)' et m ont les mêmes significations que dans la formule I, avec des amines de formules IIIa-IIIe, ou en ce que

d) on fait réagir un composé de formule VII

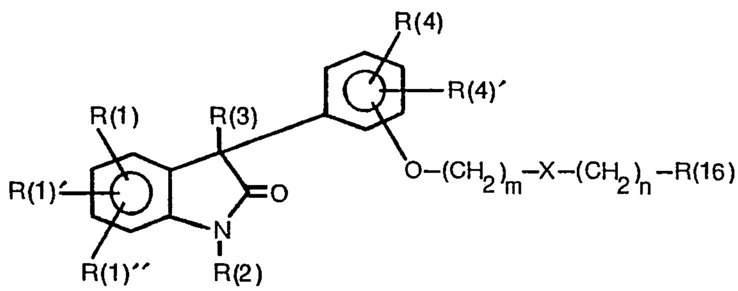

(VII)

dans laquelle R(1), R(1)', R(1)'', R(2), R(3), R(4), R(4)', X, m et n ont les mêmes significations que dans la formule I, et R(16) est l'un des groupes suivants

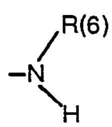

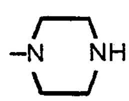 ,

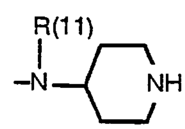 ,

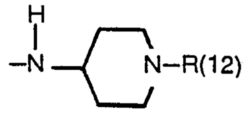 ,

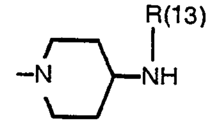

dans lesquels R(6), R(11), R(12) et R(13) ont les mêmes significations que dans la formule I, avec des agents d'alkylation ou d'acylation.

2. Procédé selon la revendication 1, caractérisé en ce que

R(1) et R(1)' sont identiques ou différents, et représentent, indépendamment l'une de l'autre, un atome d'hydrogène ou de fluor ou de chlore, ou un groupe méthyle, éthyle, méthoxy, éthoxy, $CF_3$, nitro ou acétamido;

R(1)'' représente un atome d'hydrogène;

R(2) représente un atome d'hydrogène ou un radical alkyle en $C_1—C_6$ à chaîne droite ou ramifiée, allyle, méthallyle, benzyle, phénéthyle, 4-méthoxybenzyle, 3,4-diméthoxybenzyle, 3,4,5-triméthoxybenzyle, 3,4-méthylènedioxybenzyle;

R(3) représente un atome d'hydrogène ou un radical alkyle en $C_1$—$C_{12}$ à chaîne droite ou ramifiée, allyle, méthallyle, cycloalkyle en $C_5$—$C_7$, cycloalkyl($C_5$—$C_7$)-alkyle($C_1$—$C_4$), benzyle, méthylbenzyle, fluorobenzyle, méthoxybenzyle, diméthoxybenzyle, phényléthyle;

R(4) représente un atome d'hydrogène ou de chlore, ou un groupe méthyle, méthoxy, éthoxy, nitro, hydroxy, acétamido, ou amino

R(4)' représente un atome d'hydrogène;

m est 1, 2;

n, est 0, 1, 2;

X représente le groupe $CH_2$;

R(5) représente l'un des groupes

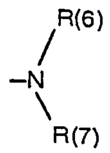

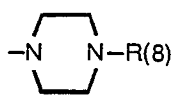

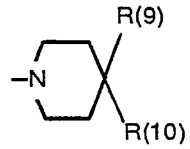

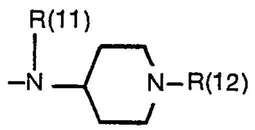

ou

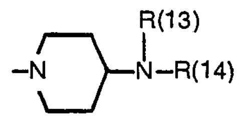

dans lesquels

R(6) représente un atome d'hydrogène ou le radical méthyle, éthyle, propyle, isopropyle;

R(7) représente un atome d'hydrogène ou le radical méthyle, éthyle, propyle, isopropyle, cyclopentyléthyle, cyclohexyléthyle, phényl-alkyle($C_1$—$C_4$), benzhydryle ou benzhydryl-alkyle($C_1$—$C_4$), les fragments phényle n'étant pas substitués ou étant substitués par un, deux ou trois substituants choisis parmi F, Cl, $CF_3$ ou des groupes alkyles en $C_1$—$C_4$, alcoxy en $C_1$—$C_4$, alkylène($C_1$—$C_2$)-dioxy ou hydroxy, ou un radical pyridyl-alkyle($C_1$—$C_4$);

R(8) représente un atome d'hydrogène ou un radical alkyle en $C_1$—$C_6$ à chaîne droite ou ramifiée, alcanoyle en $C_1$—$C_6$, phényle, le fragment phényle pouvant être substitué par un ou deux substituants choisis parmi F, Cl, $CF_3$ ou des radicaux alkyle en $C_1$—$C_4$, alcoxy en $C_1$—$C_4$, alkylène($C_1$—$C_2$)-dioxy ou hydroxy, un radical phényl-alkyle($C_1$—$C_4$), phényl-alcényle($C_3$—$C_5$), benzhydryle ou benzhydryl-alkyle($C_1$—$C_4$), phényl-alcanoyle($C_1$—$C_4$) ou benzoyle, les fragments phényle n'étant pas substitués ou étant substitués par un, deux ou trois substituants choisis parmi F, Cl, $CF_3$ ou des groupes méthyle, éthyle, méthoxy, éthoxy, alkylène($C_1$—$C_2$)-dioxy ou hydroxy;

R(9) représente un radical phényle, phényl-alkyle($C_1$—$C_4$), le fragment phényle n'étant pas substitué ou étant substitué par un, deux ou trois substituants choisis parmi F, Cl, $CF_3$ ou des groupes alkyle en $C_1$—$C_4$, alcoxy en $C_1$—$C_4$, alkylène($C_1$—$C_2$)-dioxy ou hydroxy;

R(10) représente un atome d'hydrogène ou le groupe hydroxy ou méthoxy;

R(11), R(12), R(13) et R(14) sont identiques ou différents et représentent un atome d'hydrogène ou un radical alkyle en $C_1$—$C_8$, alcanoyle en $C_1$—$C_6$, phényl-alkyle($C_1$—$C_4$), benzhydryle ou benzhydryl-alkyle($C_1$—$C_4$), les fragments phényle n'étant pas substitués ou pouvant être substitués par un, deux ou trois substituants choisis parmi F, Cl, $CF_3$ ou des groupes méthyle, éthyle, méthoxy, éthoxy, alkylène($C_1$—$C_2$)-dioxy ou hydroxy, un radical phényl-alcanoyle($C_1$—$C_4$) ou benzoyle, les fragments phényle n'étant pas substitués ou étant substitués par un, deux ou trois substituants choisis parmi F, Cl, $CF_3$ ou des groupes méthyle, éthyle, méthoxy, éthoxy, alkylène($C_1$—$C_2$)-dioxy ou hydroxy.

3. Procédé selon l'une des revendications 1 et 2, caractérisé en ce que

R(1) représente un atome d'hydrogène, de fluor ou de chlore, ou le groupe méthyle ou méthoxy;

R(1)' représente un atome d'hydrogène ou le groupe méthoxy;

R(1)'' représente un atome d'hydrogène;

R(2) représente un atome d'hydrogène ou le groupe méthyle, éthyle, propyle, isopropyle, butyle, sec-butyle, isobutyle, benzyle, phénéthyle;

R(3) représente un atome d'hydrogène ou un radical alkyle en $C_1$—$C_{12}$ à chaîne droite ou ramifiée, cyclopentyle, cyclohexyle, cyclopentylméthyle, cyclohexylméthyle, allyle, métallyle, benzyle, méthylbenzyle, fluorobenzyle, méthoxybenzyle, diméthoxybenzyle, phényléthyle;

R(4) représente un atome d'hydrogène ou de chlore ou le groupe méthoxy, méthyle, nitro ou hydroxy;

R(4)' représente un atome d'hydrogène;

m est 1, 2;

n est 0, 1 ou 2;

X représente le groupe $CH_2$;

R(5) représente l'un des groupes suivants

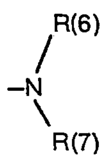

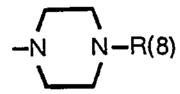

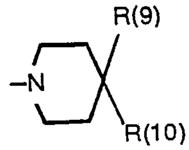

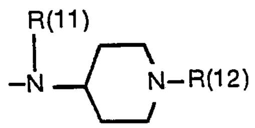

ou

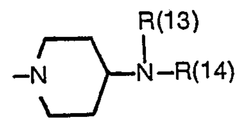

dans lesquels

R(6) représente un atome d'hydrogène ou le groupe méthyle;

R(7) représente un radical phényl-alkyle($C_1$—$C_4$), benzhydryle ou benzhydryl-alkyle($C_1$—$C_4$), le fragment phényle n'étant pas substitué ou étant substitué par un, deux ou trois substituants choisis parmi des atomes de fluor et de chlore ou les groupes méthyle, méthoxy, méthylènedioxy ou hydroxy;

R(8) représente un radical alkyle en $C_1$—$C_6$ à chaîne droite ou ramifiée, alcanoyle en $C_1$—$C_6$, phényle, phénylalkyle($C_1$—$C_4$), benzhydryle ou benzhydryl-alkyle($C_1$—$C_4$), phényl-alcanoyle($C_1$—$C_4$) ou benzoyle, les radicaux phényle n'étant pas substitués ou étant substitués par un, deux ou trois substituants choisis parmi des atomes de fluor ou de chlore ou les groupes méthyle, méthoxy, éthoxy, méthylènedioxy ou hydroxy;

R(9) représente un radical phényle, le radical phényle n'étant pas substitué ou étant substitué par un, deux ou trois substituants choisis parmi des atomes de fluor ou de chlore ou les groupes méthyle, méthoxy, méthylènedioxy ou hydroxy;

R(10) représente un atome d'hydrogène ou le groupe hydroxy ou méthoxy;

R(11), R(12), R(13) et R(14) sont identiques ou différents et représentent un atome d'hydrogène ou un radical alkyle en $C_1$—$C_6$, alcanoyle en $C_1$—$C_6$, phényl-alkyle($C_1$—$C_4$), benzhydryle ou benzhydryl-alkyle($C_1$—$C_4$), phénylalcanoyle($C_1$—$C_4$) ou benzoyle, les fragments phényle n'étant pas substitués ou étant substitués par un, deux ou trois substituants choisis parmi des atomes de fluor ou de chlore ou les groupes méthyle, méthoxy, méthylènedioxy ou hydroxy.

4. Procédé pour la préparation d'un composé de formule générale II

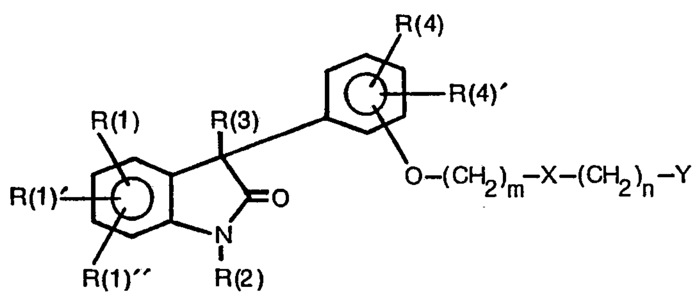

(II)

dans laquelle R(1), R(1)', R(1)'', R(2), R(3), R(4), R(4)', X, m et n ont les mêmes significations que dans la formule I et dans laquelle Y est tel que défini dans la revendication 1, caractérisé en ce que l'on fait réagir des anilines substituées de formule VIII

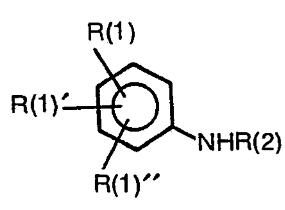

(VIII)

dans laquelle R(1), R(1)', R(1)'' et R(2) ont les mêmes significations que dans la formule I, dans laquelle

formule VIII au moins une position ortho par rapport au groupe amino doit être libre, avec un composé de formule IX

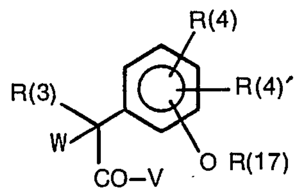

(IX)

dans laquelle R(3), R(4) et R(4)' ont les mêmes significations que dans la formule I, R(17) représente un groupe protecteur séparable dans des conditions douces, V représente OH, Cl ou un groupe O-alkyle($C_1$—$C_4$), et W représente un atome de chlore ou de brome ou le groupe OH ou OAc, selon les méthodes générales connues pour la synthèse d'amides à partir d'amines et de dérivés d'acides carboxyliques, ce par quoi il se forme des composés de formule X

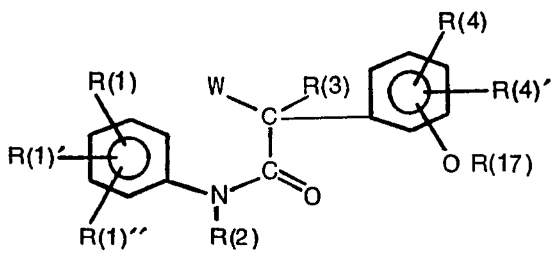

(X)

dans laquelle R(1), R(1)', R(1)'', R(2), R(3), R(4) et R(4)' ont les mêmes significations que dans la formule I, et R(17) et W ont les mêmes significations que dans la formule IX; en ce que, lorsque dans la formule X, W représente le groupe O-acétyle, on le convertit en un groupe hydroxy libre, avant la réaction de cyclisation subséquente, en ce qu l'on fait réagir les composés de formule X avec des agents de déshydratation, pour aboutir à des composés de formule XI

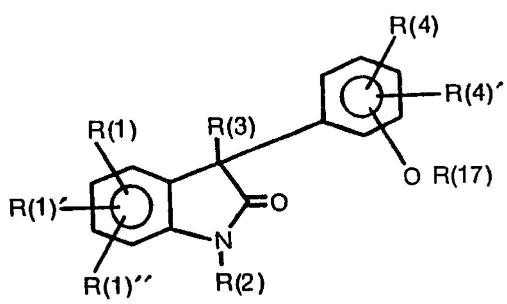

(XI)

dans laquelle R(1), R(1)', R(1)'', R(2), R(3), R(4) et R(4)' ont les mêmes significations que dans la formule I et R(17) a la même signification que dans la formule IX; en ce que l'on prépare ensuite par élimination du groupe protecteur R(17), dans des conditions convenables, les composés de formule IV

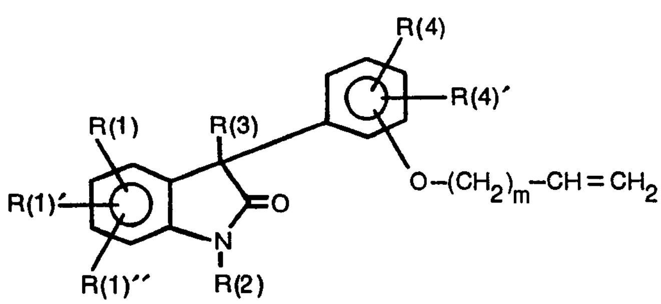

(XV)

et en ce que l'on prépare des compoés de formule II à partir de deux-ci, par réaction avec des composés de formule XIII

$$Q—(CH_2)_m—X—(CH_2)_n—Y \qquad (XIII)$$

dans laquelle X, m et n ont les mêmes significations que dans la formule I, Y a la même signification que dans la formule II, Q est défini comme Y dans la formule II, en présence de bases.

5. Procédé pour la fabrication d'un médicament pour le traitement de l'hypertension, caractérisé en ce que l'on mélange un composé I selon la revendication I avec des additifs pharmaceutiquement usuels.

**Claims for the Contracting States: BE CH DE FR GB IT LI LU NL SE**

1. A compound of the formula I

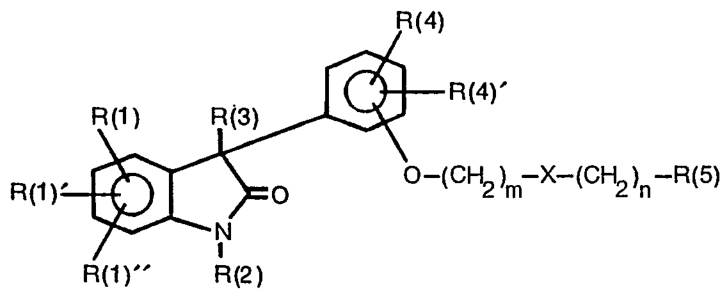

(I)

in which

R(1), R(1)' and R(1)'' are identical or different and are independent of one another and denote hydrogen, ($C_1$—$C_4$)-alkyl, ($C_1$—$C_3$)-alkoxy, F, Cl, Br, $CF_3$, nitro, hydroxyl, acetamido or amino,

R(2) denotes hydrogen, ($C_1$—$C_{10}$)-alkyl, straight-chain or branched, ($C_3$—$C_{10}$)-alkenyl, straight-chain or branched, phenyl-($C_1$—$C_4$)-alkyl, the phenyl ring being unsubstituted or substituted by one, two or three substituents from the group comprising ($C_1$—$C_4$)-alkyl, ($C_1$—$C_3$)-alkoxy, F, Cl, $CF_3$, ($C_1$—$C_2$)-alkylenedioxy or nitro,

R(3) denotes hydrogen, ($C_1$—$C_{15}$)-alkyl, straight-chain or branched, ($C_3$—$C_{15}$)-alkenyl, straight-chain or branched, ($C_4$—$C_8$)-cycloalkyl, ($C_4$—$C_8$)-cycloalkyl-($C_1$—$C_4$)-alkyl, phenyl or phenyl-($C_1$—$C_4$)-alkyl, the phenyl radical being unsubstituted or substituted by one, two or three substituents from the group comprising ($C_1$—$C_4$)-alkyl, ($C_1$—$C_3$)-alkoxy, F, Cl, $CF_3$, ($C_1$—$C_2$)-alkylenedioxy or nitro,

R(4) and R(4)' denote, identically or differently and independently of one another, hydrogen, ($C_1$—$C_4$)-alkyl, ($C_1$—$C_3$)-alkoxy, F, Cl or $CF_3$,

m denotes 1 or 2,

n denotes 0, 1 or 2,

X is a $CH_2$ group,

R(5) denotes one of the following groups

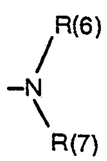

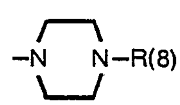

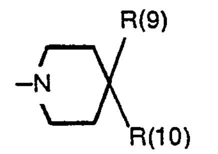

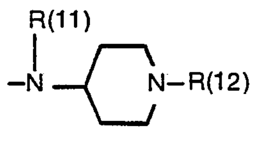

or

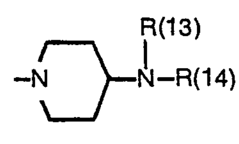

in which

R(6) and R(7) denote, identically or differently independently of one another, hydrogen, ($C_1$—$C_{10}$)-alkyl, ($C_4$—$C_8$)-cycloalkyl, ($C_4$—$C_8$)-cycloalkyl-($C_1$—$C_4$)-alkyl, pyridyl-($C_1$—$C_4$)-alkyl, phenyl-($C_1$—$C_6$)-alkyl, benzhydryl or benzhydryl-($C_1$—$C_4$)-alkyl, the phenyl radicals each being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, Br, $CF_3$ or hydroxyl,

R(8) denotes hydrogen, ($C_1$—$C_{10}$)-alkyl, straight-chain or branched, ($C_1$—$C_8$)-alkanoyl, pyridyl, pyrimidinyl, phenyl, phenyl-($C_1$—$C_4$)-alkyl, phenyl-($C_3$—$C_5$)-alkenyl, benzhydryl or benzhydryl-($C_1$—$C_4$)-alkyl, phenyl-($C_1$—$C_4$)-alkanoyl or benzoyl, the phenyl radicals each being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, Br, $CF_3$ or hydroxyl,

R(9) denotes hydrogen, ($C_1$—$C_{10}$)-alkyl, phenyl, phenyl-($C_1$—$C_4$)-alkyl, the phenyl radical in each case being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, Br, $CF_3$ or hydroxyl,

R(10) denotes hydrogen, hydroxyl or ($C_1$—$C_4$)-alkoxy, and

R(11) and R(12) or R(13) and R(14) denote, identically or differently and independently of one another, hydrogen, ($C_1$—$C_{10}$)-alkyl, straight-chain or branched, ($C_1$—$C_6$)-alkanoyl, phenyl-($C_1$—$C_4$)-alkyl, benzhydryl or benzhydryl-($C_1$—$C_4$)-alkyl, phenyl-($C_1$—$C_4$)-alkanoyl or benzoyl, the phenyl radicals each being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, Br, $CF_3$ or hydroxyl,

and the salts of the compound of the formula I with pharmaceutically acceptable acids.

2. A compound as claimed in claim 1, wherein

R(1) and R(1)' are identical or different and denote, independently of one another, hydrogen, methyl, ethyl, methoxy, ethoxy, fluorine, chlorine, $CF_3$, nitro or acetamido,

R(1)'' denotes hydrogen

R(2) denotes hydrogen, ($C_1$—$C_6$)-alkyl, straight-chain or branched, allyl, methallyl, benzyl, phenethyl, 4-methoxybenzyl, 3,4-dimethoxybenzyl, 3,4,5-trimethoxybenzyl, 3,4-methylenedioxybenzyl,

R(3) denotes hydrogen, ($C_1$—$C_{12}$)-alkyl, straight-chain or branched, allyl, methallyl, ($C_5$—$C_7$)-cycloalkyl, ($C_5$—$C_7$)-cycloalkyl-($C_1$—$C_4$)-alkyl, benzyl, methylbenzyl, fluorobenzyl, methoxybenzyl, dimethoxybenzyl, phenylethyl,

R(4) denotes hydrogen, methyl, methoxy, ethoxy, chlorine,

R(4)' denotes hydrogen,

m denotes 1 or 2,

n denotes 0, 1 or 2,

X denotes a $CH_2$ group,

R(5) denotes one of the groups

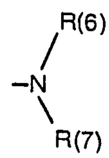

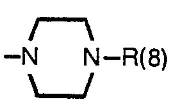

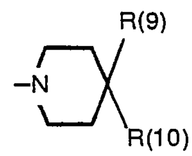

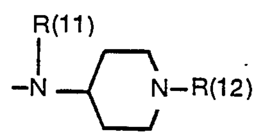

or

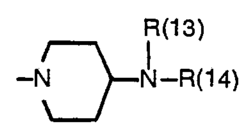

in which

R(6) denotes hydrogen, methyl, ethyl, propyl, isopropyl,

R(7) denotes hydrogen, methyl, ethyl, propyl, isopropyl, cyclopentylethyl, cyclohexylethyl, phenyl-($C_1$—$C_4$)-alkyl, benzhydryl or benzhydryl-($C_1$—$C_4$)-alkyl, the phenyl radicals each being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, $CF_3$ or hydroxyl, or pyridyl-($C_1$—$C_4$)-alkyl,

R(8) denotes hydrogen, ($C_1$—$C_6$)-alkyl, straight-chain or branced, ($C_1$—$C_6$)-alkanoyl, phenyl, it being possible for the phenyl radical to be substituted by one or two radicals from the group comprising ($C_1$—$C_4$)-alkyl, ($C_1$—$C_4$)-alkoxy, ($C_1$—$C_2$)-alkylenedioxy, F, Cl, $CF_3$ or hydroxyl, or denotes phenyl-($C_1$—$C_4$)-alkyl, phenyl-($C_3$—$C_5$)-alkenyl, benzhydryl or benzhydryl-($C_1$—$C_4$)-alkyl, phenyl-($C_1$—$C_4$)-alkanoyl or benzoyl, the phenyl radicals each being unsubstituted or substituted by one, two or three radicals from the group comprising methyl, ethyl, methoxy, ethoxy, ($C_1$—$C_2$)-alkylenedioxy, F, Cl, $CF_3$ or hydroxyl,

R(9) denotes phenyl, phenyl-($C_1$—$C_4$)-alkyl, the phenyl radical in each case being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, $CF_3$ or hydroxyl,

R(10) denotes hydrogen, hydroxyl or methoxy,

R(11), R(12), (R13) and R(14) identical or different and denote hydrogen, ($C_1$—$C_8$)-alkyl, ($C_1$—$C_6$)-alkanoyl, phenyl-($C_1$—$C_4$)-alkyl, benzhydryl or benzhydryl-($C_1$—$C_4$)-alkyl, it being possible for the phenyl radicals each to be unsubstituted or substituted by one, two or three radicals from the group comprising

methyl, ethyl, methoxy, ethoxy, $(C_1—C_2)$-alkylenedioxy, F, Cl, $CF_3$ or hydroxyl, or denotes phenyl-$(C_1—C_4)$-alkanoyl or benzoyl, the phenyl radicals each being unsubstituted or substituted by one, two or three radicals from the group comprising methyl, ethyl, methoxy, ethoxy, $(C_1—C_2)$-alkylenedioxy, F, Cl, $CF_3$ or hydroxyl,

and the salts of these compounds of the formula I with pharmaceutically acceptable acids.

3. A compound as claimed in one of claims 1 and 2, wherein

R(1) denotes hydrogen, methyl, methoxy, fluorine or chlorine,

R(1)' denotes hydrogen or methoxy,

R(1)'' denotes hydrogen,

R(2) denotes hydrogen, methyl, ethyl, propyl, isopropyl, butyl, sec. butyl, isobutyl, benzyl, phenethyl,

R(3) denotes hydrogen, $(C_1—C_{12})$-alkyl, straight-chain or branched, cyclopentyl, cyclohexyl, cyclopentylmethyl, cyclohexylmethyl, allyl, methallyl, benzyl, methylbenzyl, fluorobenzyl, methoxybenzyl, dimethoxybenzyl, phenylethyl,

R(4) denotes hydrogen, methoxy, methyl,

R(4)' denotes hydrogen,

m denotes 1 or 2,

n denotes 0, 1 or 2,

X denotes a $CH_2$ group,

R(5) denotes one of the following groups

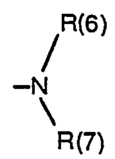

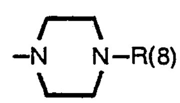

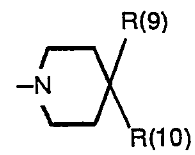

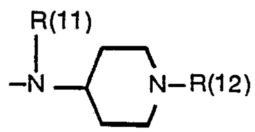

or

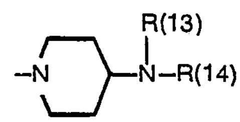

in which

R(6) denotes hydrogen or methyl,

R(7) denotes phenyl-$(C_1—C_4)$-alkyl, benzhydryl or benzhydryl-$(C_1—C_4)$-alkyl, the phenyl radical in each case being unsubstituted or substituted by one, two or three radicals from the group comprising methyl, methoxy, fluorine, chlorine, methylenedioxy or hydroxyl,

R(8) denotes $(C_1—C_6)$-alkyl, straight-chain or branched, $(C_1—C_6)$-alkanoyl, phenyl, phenyl-$(C_1—C_4)$-alkyl, benzhydryl or benzhydryl-$(C_1—C_4)$-alkyl, phenyl-$(C_1—C_4)$-alkanoyl or benzoyl, the phenyl radicals each being unsubstituted or substituted by one, two or three radicals from the group comprising methyl, methoxy, ethoxy, methylenedioxy, fluorine, chlorine or hydroxyl,

R(9) denotes phenyl, the phenyl radical being unsubstituted or substituted by one, two or three radicals from the group comprising methyl, methoxy, fluorine, chlorine, methylenedioxy or hydroxyl,

R(10) denotes hydrogen, hydroxyl or methoxy,

R(11), R(12), R(13) and R(14) are identical or different and denote hydrogen, $(C_1—C_6)$-alkyl, $(C_1—C_6)$-alkanoyl, phenyl-$(C_1—C_4)$-alkyl, benzhydryl or benzhydryl-$(C_1—C_4)$-alkyl, phenyl-$(C_1—C_4)$-alkanoyl or benzoyl, the phenyl radicals each being unsubstituted or substituted by one, two or three radicals from the group comprising methyl, methoxy, methylenedioxy, fluorine, chlorine or hydroxyl,

and the salts of these compounds of the formula I with pharmaceutically acceptable acids.

4. A process for the preparation of a compound of the formula I as claimed in claim 1, which comprises

a) reaction of a compound of the formula II

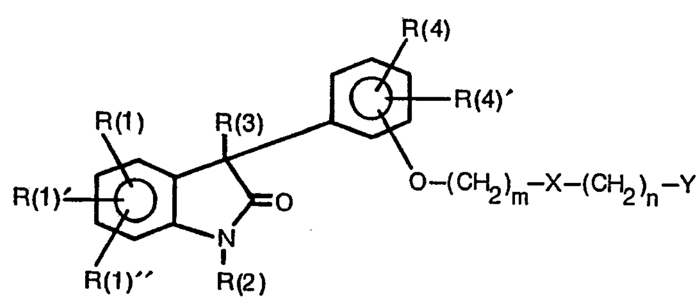

(II)

in which (R1), R(1)', R(1)'', R(2), R(3), R(4), R(4)', X, m and n have the same meaning as in formula I, and in which Y is a chlorine, bromine or iodine atom, or a sulfonic acid, toluenesulfonyl or trifluoromethanesulfonyl radical, with one of the compounds of the formulae IIIa, IIIb, IIIc, IIId or IIIe

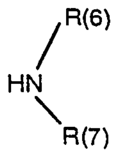

IIIa

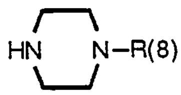

IIIb

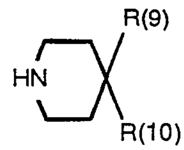

IIIc

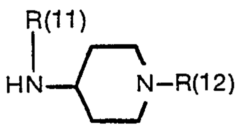

IIId

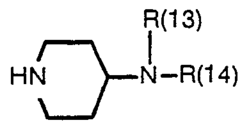

IIIe

in which R(6), R(7), R(8), R(9), R(10), R(11), R(12), R(13) and R(14) have the same meaning as in formula I, under the conditions of a nucleophilic substitution, or

b) reaction of a compound of the formula IV

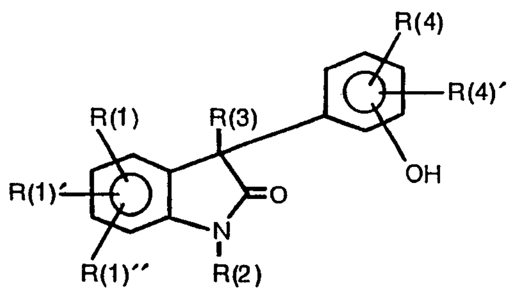

(IV)

in which R(1), R(1)', R(1)'', R(2), R(3), R(4) and R(4)' have the same meaning as in formula I, with a compound of the formula V

$$Z—(CH_2)_m—X—(CH_2)_n—R(5) \qquad V$$

in which Z has the same definition as Y in formula II, and in which R(5), X, m and n have the same meaning as in formula I, or

c) reaction of a compound of the formula VI

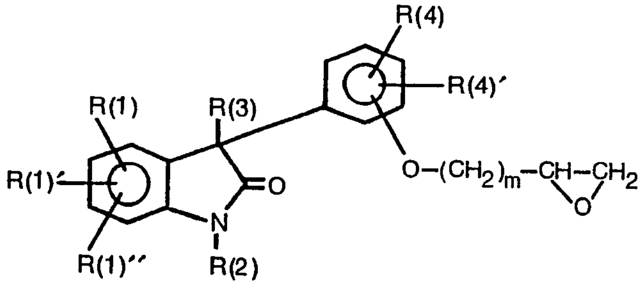

(VI)

in which R(1), R(1)', R(1)'', R(2), R(3), R(4), R(4)' and m have the same meaning as in formula I, with amines of the formula IIIa—IIIe or

d) reaction of a compound of the formula VII

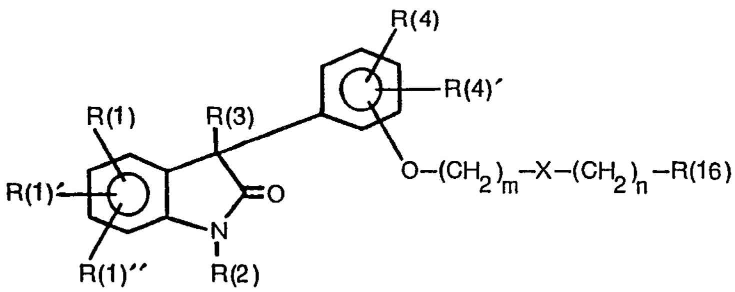

(VII)

in which R(1), R(1)', R(1)'', R(2), R(3), R(4), R(4)', X, m and n have the same meaning as in formula I, and R(16) denotes one of the following groups

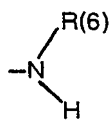

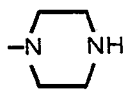 ,

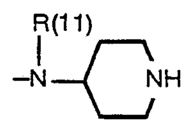 ,

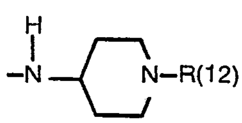 ,

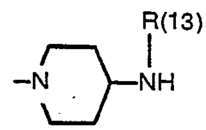

in which R(6), R(11), R(12) and R(13) have the same meaning as in formula I, with alkylating or acylating agents.

5. A compound of the general formula II

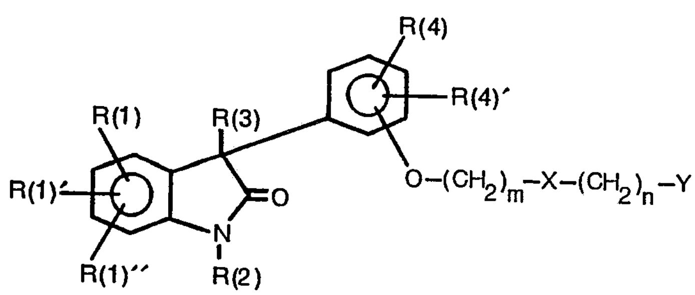

(II)

in which R(1), R(1)', R(1)'', R(2), R(3), R(4), R(4)', X, m and n have the same meaning as in formula I, and in which Y is a chlorine, bromine or iodine atom, a sulfonic acid radical, a toluenesulfonyl radical or a trifluoromethanesulfonyl radical.

6. The use of a compound I as claimed in claim 1 for the preparation of an agent which lowers blood pressure.

7. The use of a compound I as claimed in claim 1 as an agent to lower blood pressure.

**Claims for the Contracting State: AT**

1. A process for the preparation of a compound of the formula I

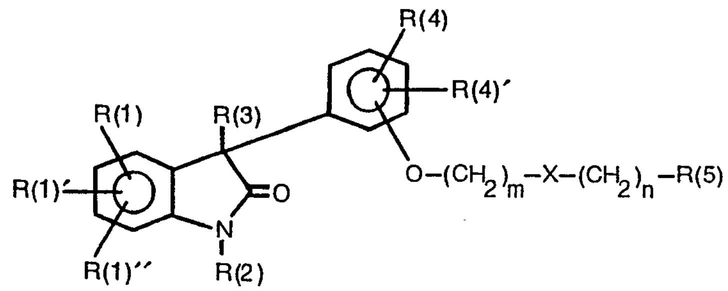

(I)

in which

R(1), R(1)' and R(1)'' are identical or different and are independent of one another and denote hydrogen, $(C_1—C_4)$-alkyl, $(C_1—C_3)$-alkoxy, F, Cl, Br, $CF_3$, nitro, hydroxyl, acetamido or amino,

R(2) denotes hydrogen, $(C_1—C_{10})$-alkyl, straight-chain or branched, $(C_3—C_{10})$-alkenyl, straight-chain or branched, phenyl-$(C_1—C_4)$-alkyl, the phenyl ring being unsubstituted or substituted by one, two or three substituents from the group comprising $(C_1—C_4)$-alkyl, $(C_1—C_3)$-alkoxy, F, Cl, $CF_3$, $(C_1—C_2)$-alkylenedioxy or nitro,

R(3) denotes hydrogen, $(C_1—C_{15})$-alkyl, straight-chain or branched, $(C_3—C_{15})$-alkenyl, straight-chain or branched, $(C_4—C_8)$-cycloalkyl, $(C_4—C_8)$-cycloalkyl-$(C_1—C_4)$-alkyl, phenyl or phenyl-$(C_1—C_4)$-alkyl, the phenyl radical being unsubstituted or substituted by one, two or three substituents from the group comprising $(C_1—C_4)$-alkyl, $(C_1—C_3)$-alkoxy, F, Cl, $CF_3$, $(C_1—C_2)$-alkylenedioxy or nitro,

R(4) and R(4)' denote, identically or differently and independently of one another, hydrogen, $(C_1—C_4)$-alkyl, $(C_1—C_3)$-alkoxy, F, Cl, $CF_3$, nitro, hydroxyl, acetamido or amine,

m denotes 1 or 2,

n denotes 0, 1 or 2,

X is a $CH_2$ group,

R(5) denotes one of the following groups

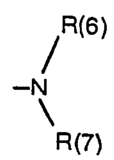

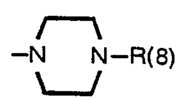

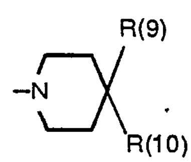

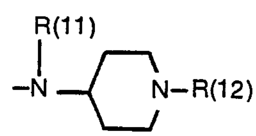

or

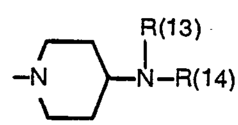

in which

R(6) and R(7) denote, identically or differently independently of one another, hydrogen, $(C_1—C_{10})$-alkyl, $(C_4—C_8)$-cycloalkyl, $(C_4—C_8)$-cycloalkyl-$(C_1—C_4)$-alkyl, pyridyl-$(C_1—C_4)$-alkyl, phenyl-$(C_1—C_6)$-alkyl, benzhydryl or benzhydryl-$(C_1—C_4)$-alkyl, the phenyl radicals each being unsubstituted or substituted by one, two or three radicals from the group comprising $(C_1—C_4)$-alkyl, $(C_1—C_4)$-alkoxy, $(C_1—C_2)$-alkylenedioxy, F, Cl, Br, $CF_3$ or hydroxyl,

R(8) denotes hydrogen, $(C_1—C_{10})$-alkyl, straight-chain or branched, $(C_1—C_8)$-alkanoyl, pyridyl, pyrimidinyl, phenyl, phenyl-$(C_1—C_4)$-alkyl, phenyl-$(C_3—C_5)$-alkenyl, benzhydryl or benzhydryl-$(C_1—C_4)$-alkyl, phenyl-$(C_1—C_4)$-alkanoyl or benzoyl, the phenyl radicals each being unsubstituted or substituted by one, two or three radicals from the group comprising $(C_1—C_4)$-alkyl, $(C_1—C_4)$-alkoxy, $(C_1—C_2)$-alkylenedioxy, F, Cl, Br, $CF_3$ or hydroxyl,

R(9) denotes hydrogen, $(C_1—C_{10})$-alkyl, phenyl, phenyl-$(C_1—C_4)$-alkyl, the phenyl radical in each case being unsubstituted or substituted by one, two or three radicals from the group comprising $(C_1—C_4)$-alkyl, $(C_1—C_4)$-alkoxy, $(C_1—C_2)$-alkylenedioxy, F, Cl, Br, $CF_3$ or hydroxyl,

R(10) denotes hydrogen, hydroxyl or ($C_1$—$C_4$)-alkoxy, and

R(11) and R(12) or R(13) and R(14) denote, identically or differently and independently of one another, hydrogen, ($C_1$—$C_{10}$)-alkyl, straight-chain or branched, ($C_1$—$C_6$)-alkanoyl, phenyl-($C_1$—$C_4$)-alkyl, benzhydryl or benzhydryl-($C_1$—$C_4$)-alkyl, phenyl-($C_1$—$C_4$)-alkanoyl or benzoyl, the phenyl radicals each being unsubstituted or substituted by one, two or three radicals from the group comprising ($C_1$—$C_4$)-alkyl, ($C_1$—$C_4$)-alkoxy, ($C_1$—$C_2$)-alkylenedioxy, F, Cl, Br, $CF_3$ or hydroxyl,

and the salts of the compound of the formula I with pharmaceutically acceptable acids, which comprises

a) reaction of a compound of the formula II

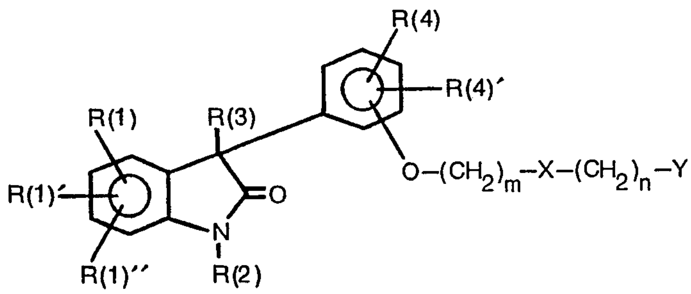

(II)

in which (R1), R(1)', R(1)'', R(2), R(3), R(4), R(4)', X, m and n have the same meaning as in formula I, and in which Y is a chlorine, bromine or iodine atom, or a sulfonic acid, toluenesulfonyl or trifluoromethanesulfonyl radical, with one of the compounds of the formulae IIIa, IIIb, IIIc, IIId or IIIe

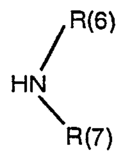

IIIa

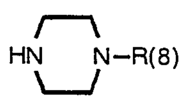

IIIb

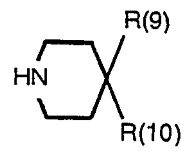

IIIc

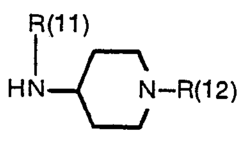

IIId

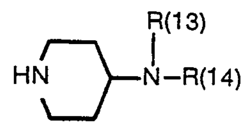

IIIe

in which R(6), R(7), R(8), R(9), R(10), R(11), R(12), R(13) and R(14) have the same meaning as in formula I, under the conditions of a nucleophilic substitution, or

b) reaction of a compound of the formula IV

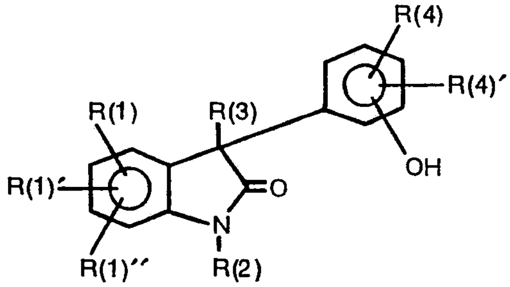

(IV)

in which R(1), R(1)', R(1)'', R(2), R(3), R(4) and R(4)' have the same meaning as in formula I, with a compound of the formula V

$$Z—(CH_2)_m—X—(CH_2)_n—R(5) \qquad V$$

in which Z has the same definition as Y in formula II, and in which R(5), X, m and n have the same meaning as in formula I, or

c) reaction of a compound of the formula VI

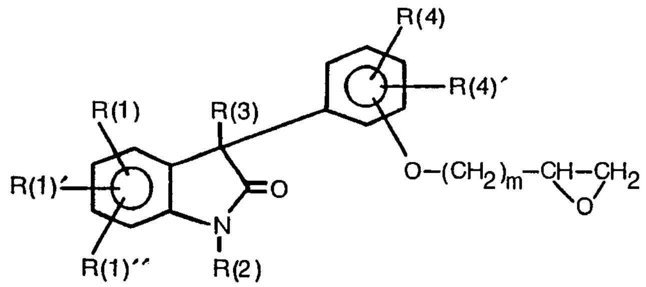

(VI)

in which R(1), R(1)', R(1)'', R(2), R(3), R(4), R(4)' and m have the same meaning as in formula I, with amines of the formula IIIa—IIIe or

d) reaction of a compound of the formula VII

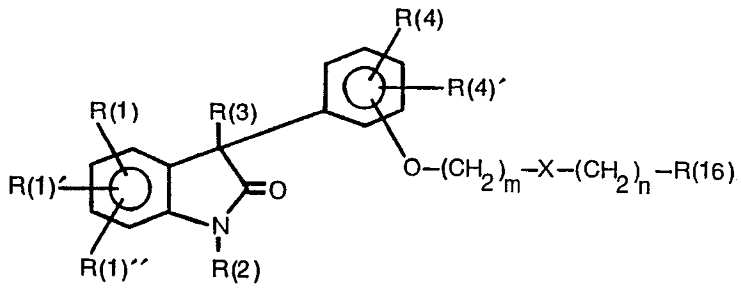

(VII)

in which R(1), R(1)', R(1)'', R(2), R(3), R(4), R(4)', X, m and n have the same meaning as in formula I, and R(16) denotes one of the following groups

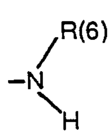 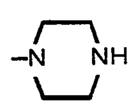 , 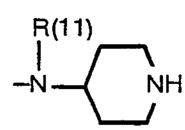 ,

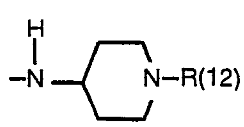 , 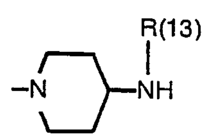

in which R(6), R(11), R(12) and R(13) have the same meaning as in formula I, with alkylating or acylating agents.

2. A process as claimed in claim 1, wherein

R(1) and R(1)' are identical or different and denote, independently of one another, hydrogen, methyl, ethyl, methoxy, ethoxy, fluorine, chlorine, $CF_3$, nitro or acetamido,

R(1)'' denotes hydrogen

R(2) denotes hydrogen, $(C_1—C_6)$-alkyl, straight-chain or branched, allyl, methallyl, benzyl, phenethyl, 4-methoxybenzyl, 3,4-dimethoxybenzyl, 3,4,5-trimethoxybenzyl, 3,4-methylenedioxybenzyl,

R(3) denotes hydrogen, $(C_1—C_{12})$-alkyl, straight-chain or branched, allyl, methallyl, $(C_6—C_7)$-cycloalkyl, $(C_5—C_7)$-cycloalkyl-$(C_1—C_4)$-alkyl, benzyl, methylbenzyl, fluorobenzyl, methoxybenzyl, dimethoxybenzyl, phenylethyl,

R(4) denotes hydrogen, methyl, methoxy, ethoxy, chlorine, nitro, hydroxyl, acetamido or amino,

R(4)′ denotes hydrogen,

m denotes 1 or 2,

n denotes 0, 1 or 2,

X denotes a $CH_2$ group,

R(5) denotes one of the groups

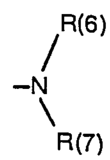
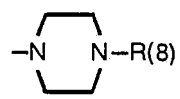
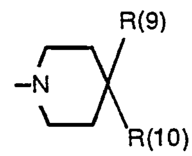

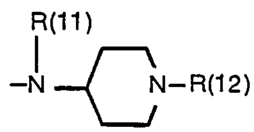
or
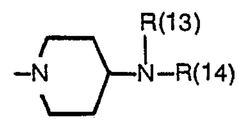

in which

R(6) denotes hydrogen, methyl, ethyl, propyl, isopropyl,

R(7) denotes hydrogen, methyl, ethyl, propyl, isopropyl, cyclopentylethyl, cyclohexylethyl, phenyl-$(C_1—C_4)$-alkyl, benzhydryl or benzhydryl-$(C_1—C_4)$-alkyl, the phenyl radicals each being unsubstituted or substituted by one, two or three radicals from the group comprising $(C_1—C_4)$-alkyl, $(C_1—C_4)$-alkoxy, $(C_1—C_2)$-alkylenedioxy, F, Cl, $CF_3$ or hydroxyl, or pyridyl-$(C_1—C_4)$-alkyl,

R(8) denotes hydrogen, $(C_1—C_6)$-alkyl, straight-chain or branched, $(C_1—C_6)$-alkanoyl, phenyl, it being possible for the phenyl radical to be substituted by one or two radicals from the group comprising $(C_1—C_4)$-alkyl, $(C_1—C_4)$-alkoxy, $(C_1—C_2)$-alkylenedioxy, F, Cl, $CF_3$ or hydroxyl, or denotes phenyl-$(C_1—C_4)$-alkyl, phenyl-$(C_3—C_5)$-alkenyl, benzhydryl or benzhydryl-$(C_1—C_4)$-alkyl, phenyl-$(C_1—C_4)$-alkanoyl or benzoyl, the phenyl radicals each being unsubstituted or substituted by one, two or three radicals from the group comprising methyl, ethyl, methoxy, ethoxy, $(C_1—C_2)$-alkylenedioxy, F, Cl, $CF_3$ or hydroxyl,

R(9) denotes phenyl, phenyl-$(C_1—C_4)$-alkyl, the phenyl radical in each case being unsubstituted or substituted by one, two or three radicals from the group comprising $(C_1—C_4)$-alkyl, $(C_1—C_4)$-alkoxy, $(C_1—C_2)$-alkylenedioxy, F, Cl, $CF_3$ or hydroxyl,

R(10) denotes hydrogen, hydroxyl or methoxy,

R(11), R(12), (R13) and R(14) identical or different and denote hydrogen, $(C_1—C_8)$-alkyl, $(C_1—C_6)$-alkanoyl, phenyl-$(C_1—C_4)$-alkyl, benzhydryl or benzhydryl-$(C_1—C_4)$-alkyl, it being possible for the phenyl radicals each to be unsubstituted or substituted by one, two or three radicals from the group comprising methyl, ethyl, methoxy, ethoxy, $(C_1—C_2)$-alkylenedioxy, F, Cl, $CF_3$ or hydroxyl, or denotes phenyl-$(C_1—C_4)$-alkanoyl or benzoyl, the phenyl radicals each being unsubstituted or substituted by one, two or three radicals from the group comprising methyl, ethyl, methoxy, ethoxy, $(C_1—C_2)$-alkylenedioxy, F, Cl, $CF_3$ or hydroxyl.

3. The process as claimed in one of claims 1 and 2, wherein

R(1) denotes hydrogen, methyl, methoxy, fluorine or chlorine,

R(1)′ denotes hydrogen or methoxy,

R(1)″ denotes hydrogen,

R(2) denotes hydrogen, methyl, ethyl, propyl, isopropyl, butyl, sec. butyl, isobutyl, benzyl, phenethyl,

R(3) denotes hydrogen, $(C_1—C_{12})$-alkyl, straight-chain or branched, cyclopentyl, cyclohexyl, cyclopentylmethyl, cyclohexylmethyl, allyl, methallyl, benzyl, methylbenzyl, fluorobenzyl, methoxybenzyl, dimethoxybenzyl, phenylethyl,

R(4) denotes hydrogen, methoxy, methyl, chlorine, nitro or hydroxyl

R(4)′ denotes hydrogen,

m denotes 1 or 2,

n denotes 0, 1 or 2,

X denotes a $CH_2$ group,

R(5) denotes one of the following groups

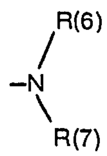 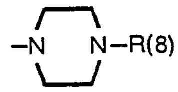 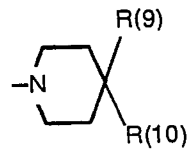

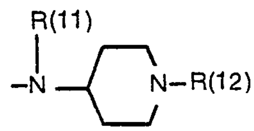 or 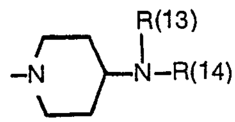

in which

R(6) denotes hydrogen or methyl,

R(7) denotes phenyl-($C_1$—$C_4$)-alkyl, benzhydryl or benzhydryl-($C_1$—$C_4$)-alkyl, the phenyl radical in each case being unsubstituted or substituted by one, two or three radicals from the group comprising methyl, methoxy, fluorine, chlorine, methylenedioxy or hydroxyl,

R(8) denotes ($C_1$—$C_6$)-alkyl, straight-chain or branched, ($C_1$—$C_6$)-alkanoyl, phenyl, phenyl-($C_1$—$C_4$)-alkyl, benzhydryl or benzhydryl-($C_1$—$C_4$)-alkyl, phenyl-($C_1$—$C_4$)-alkanoyl or benzoyl, the phenyl radicals each being unsubstituted or substituted by one, two or three radicals from the group comprising methyl, methoxy, ethoxy, methylenedioxy, fluorine, chlorine or hydroxyl,

R(9) denotes phenyl, the phenyl radical being unsubstituted or substituted by one, two or three radicals from the group comprising methyl, methoxy, fluorine, chlorine, methylenedioxy or hydroxyl,

R(10) denotes hydrogen, hydroxyl or methoxy,

R(11), R(12), R(13) and R(14) are identical or different and denote hydrogen, ($C_1$—$C_6$)-alkyl, ($C_1$—$C_6$)-alkanoyl, phenyl-($C_1$—$C_4$)-alkyl, benzhydryl or benzhydryl-($C_1$—$C_4$)-alkyl, phenyl-($C_1$—$C_4$)-alkanoyl or benzoyl, the phenyl radicals each being unsubstituted or substituted by one, two or three radicals from the group comprising methyl, methoxy, methylenedioxy, fluorine, chlorine or hydroxyl.

4. A process for the preparation of a compound of the general formula II

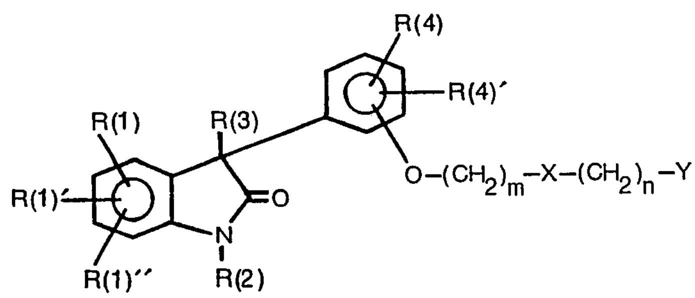

(II)

in which R(1), R(1)', R(1)'', R(2), R(3), R(4), R(4)', X, m and n have the same meaning as in formula I, and in which Y is defined as in claim 1, which comprises reaction of substituted anilines of the formula VIII

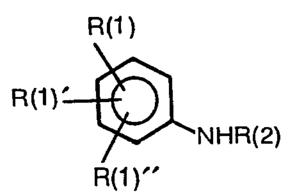

(VIII)

in which R(1), R(1)', R(1)'' and R(2) have the same meaning as in formula I, in which formula VIII at least one position ortho to the amino group must be free, with a compound of the formula IX

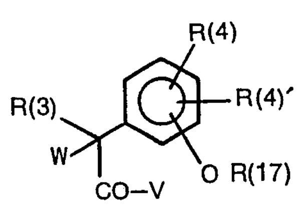

(IX)

in which R(3), R(4) and R(4)' have the same meaning as in formula I, R(17) represents a protective group which can be eliminated under mild conditions, V denotes OH, Cl or O-($C_1$—$C_4$)-alkyl, and W denotes OH, OAc, chlorine or bromine, by the generally known methods for the synthesis of amides from amines and carboxylic acid derivatives, thee being formation of compounds of the formula X

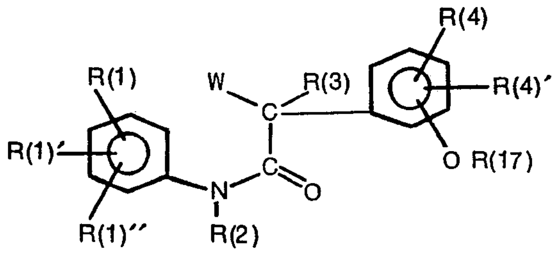

(X)

in which R(1), R(1)', R(1)'', R(2), R(3), R(4) and R(4)' have the same meaning as in formula I, R(17) and W have the same meaning as in formula IX, and comprises, where W in formula X denotes a O-acetyl group, conversion of the latter, before the subsequent cyclization reaction, into a free hydroxyl group, and comprises reaction then of the compounds of the formula X with dehydrating agents to give compounds of the formula XI

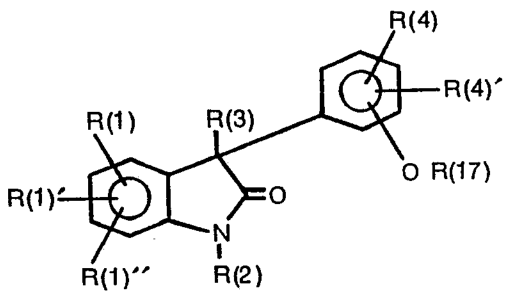

(XI)

in which R(1), R(1)', R(1)'', R(2), R(3), R(4) and R(4)' have the same meaning as in formula I, and R(17) has the same meaning as in formula IX, and comprises subsequent preparation of the compounds of the formula IV

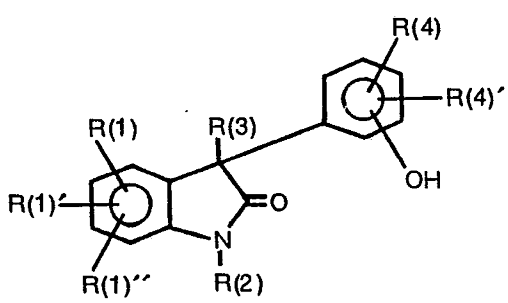

(IV)

by elimination of the protective group R(17) under suitable conditions, and comprises preparation from the latter of compounds of the formula II by reaction with compounds of the formula XIII

$$Q—(CH_2)_m—X—(CH_2)_n—Y \qquad XIII$$

in which X, m and n have the same meaning as in formula I, Y has the same meaning as in formula II, and Q has the same definition as Y in formula II, in the presence of the bases.

5. A process for the preparation of a medicament for the treatment of high blood pressure, which comprises mixing a compound I as claimed in claim 1 with pharmaceutically customary additives.